# EUROPEAN PATENT SPECIFICATION

(11) **EP 2 753 674 B1**
(45) Date of publication and mention of the grant of the patent: **10.02.2016**
(21) Application number: 12750585.7
(22) Date of filing: 17.08.2012
(51) Int. Cl.: C09K 11/06, G01N 33/58, C07D 213/40

(54) **LUMINESCENT LANTHANIDE CHELATES HAVING THREE CHROMOPHORES AND THEIR USE**
LUMINESZIERENDE LANTHANIDCHELATE MIT DREI CHROMOPHOREN UND IHRE VERWENDUNG
CHÉLATES DE LANTHANIDES LUMINESCENTS COMPRENANT TROIS CHROMOPHORES ET LEUR UTILISATION

(30) Priority: 19.08.2011 DK 201100630; 22.12.2011 DK 201100996
(43) Date of publication of application: 16.07.2014
(73) Proprietor: DHR Finland Oy, 20750 Turku (FI)
(72) Inventor: TAKALO, Harri, 20360 Turku (FI); MELTOLA, Niko, 20760 Piispanristi (FI)
(74) Representative: Marnaes, Lene Meineche
(86) International application number: PCT/EP2012/066082
(87) International publication number: WO 2013/026790

(56) References cited:
- EP-A1- 0 967 205
- EP-A2- 1 447 666
- WO-A1-00/48990
- WO-A1-00/48991
- WO-A1-2006/026038
- WO-A1-2008/020113

## Description

### FIELD OF INVENTION

The present invention relates to novel three-chromophore-containing lanthanide chelates to be attached to a biospecific reactant and their use in various assays.

### BACKGROUND OF INVENTION

Time-resolved fluorometry (TRF) employing long-lifetime emitting luminescent lanthanide chelates has been applied in many specific binding assays, such as e.g. immunoassays, DNA hybridization assays, receptor-binding assays, enzymatic assays, bio-imaging such as immunocytochemical, immunohistochemical assays or cell based assays to measure wanted analyte at very low concentration. Moreover, lanthanide chelates have been used in magnetic resonance imaging (MRI) and position emission tomography (PET)

For TRF application, an optimal label has to fulfill several requirements. First, it has to be photochemically stable both in the ground state and in the excited state and it has to be kinetically and chemically stable. The excitation wavelength has to be as high as possible, preferable over 300 nm. It has to have efficient cation emission i.e. high luminescence yield (excitation coefficient x quantum yield, εΦ). The observed luminescence decay time has to be long, and the chelate has to have good water solubility. For the purpose of labeling, it should have a reactive group to allow covalent attachment to a biospecific binding reactant, and the affinity and nonspecific binding properties of the labeled biomolecules have to be retained.

The challenge is to prepare a chelate label to fulfill all requirements in one molecule, and therefore, certain compromises are generally made in the development of suitable labels. As a consequence hereof, a number of attempts (see e.g. the review in Bioconjugate Chem., 20 (2009) 404) have been made to tune the photo-physical properties of the chelate labels suitable for time-resolved fluorometric applications.

One generally used method to improve luminescence intensity is to prepare chelate ligands with several independent chromophoric moieties combined in structure designs, which offer high stabilities and luminescence quantum yields. Chelates which contain two and three separate 4-(phenylethynyl)pyridines are published by Takalo, H., et al., 1996, Helv. Chim.Acta., 79, 789. More recent examples of lanthanide chelates and chelating ligands are those disclosed in e.g. EP 1 447 666, WO 2010/055207, WO 2010/006605 and WO 2008/020113. Based on chelate stability studies with cyclic azamacrocycles (such as DOTA), higher stabilities over open chain chelates (such as DPTA) has been observed, and thus, the main focus with disclosed chelates with three chromophores has been on azamacrocycles tethered to the various chromophores.

Lately, high lanthanide chelate stabilities have been observed with open chain ligands utilizing several 1-hydroxy-2-pyridinone and salisylamide groups although these disclosed chelates are normally eight dentate and do not contain carboxylic acids for coordination to a lanthanide ion, high luminescence and stabilities have been obtained. However, those lanthanide chelates have only moderate total molar absorptivity i.e. below 27,000 with four chromophores, whereas e.g. chelates with only one phenylethynylpyridine subunit normally have absorptivities of 25,000-35,000 cm-¹ (Latva, M, et al., 1997, J. Luminescence, 75, 149) depending on the substituents in the chromophore.

A well-known challenge with chelates and ligands having many chromophores is to find out a suitable structure design, which offers high water solubility and at the same time being inert towards any possible bioprocesses. It is known, that the addition of chromophores decreases the solubility of ligands and chelates in water, increases the formation of biospecific binding reactant aggregates during the labeling process and non-specific binding properties of labeled biomolecules. Aggregates will produce purification problems and reduced yield of labeled material. Moreover, increased non-specific binding of labeled biomolecule will enhance back-ground luminescence of biospecific assays and thus reduces assay sensitivity.

### SUMMARY OF INVENTION

The invention relates to a novel label chelate design having three individual chromophors around an emitting lanthanide ion and giving exceptionally high signal level.

With the chelates of the invention it is possible to decrease the labelling degree without losing signal, and at the same time the lower degree of labelling will improve the affinity of the biomolecule and decrease unspecific binding during the assay. Thus faster kinetic is possible and lower background is seen which can also improve the overall assay sensitivity.

A first aspect of the invention relates to a luminescent lanthanide chelate formula (I)

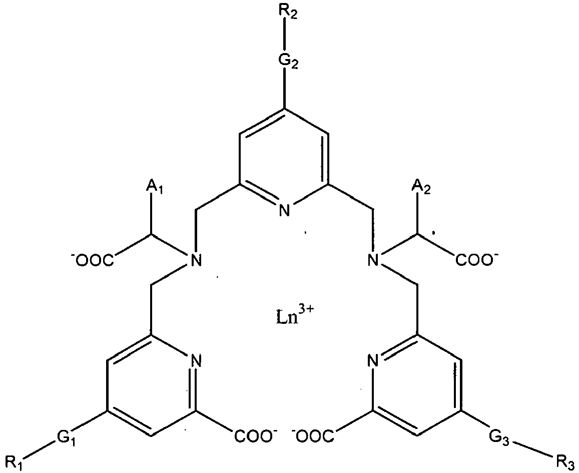

A second aspect of the invention relates to a detectable molecule comprising a biospecific binding reactant conjugated to a luminescent lanthanide chelate of the formula (I) as defined herein.

A third aspect of the invention relates to a luminescent lanthanide chelating ligand of formula (II)

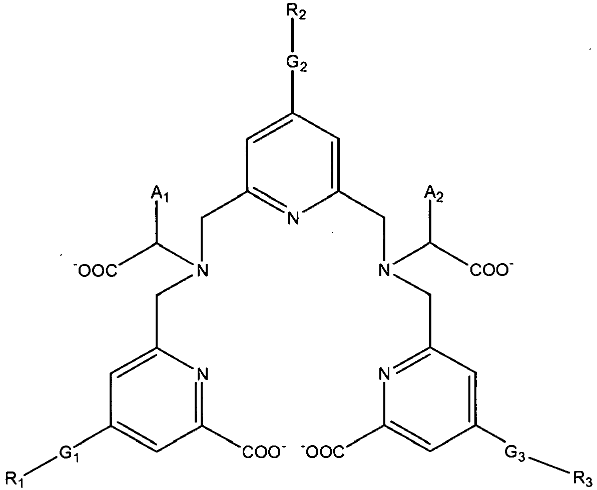

A fourth aspect of the invention relates to a method of carrying out a biospecific binding assay, said method comprising the steps of: a) forming a biocomplex between an analyte and a biospecific binding reactant labelled by the lanthanide chelate as defined herein,; b) exciting said biocomplex with radiation having an excitation wavelength, thereby forming an excited biocomplex; and c) detecting emission radiation emitted from said excited biocomplex.

A fifth aspect of the invention relates to the use of a detectable molecule as defined herein in a specific bioaffinity based binding assay utilizing time-resolved fluorometric determination of a specific luminescence.

A sixth aspect of the invention relates to a solid support material conjugated with a luminescent lanthanide chelate of the formula (I) as defined herein.

### BRIEF DESCRIPTION OF THE FIGURES

Figure 1 shows the excitation maxima of chelates **46, 48** and **75**.
Figure 2 shows the excitation maxima of chelates **19**, **47**, **59** and **60**.

### DETAILED DESCRIPTION OF INVENTION

The aim of the present invention is to provide means to obtain improved lanthanide chelate labels to be used in specific bioaffinity based binding assays, such as immunoassays (both homogeneous and heterogeneous), nucleic acid hybridization assays, receptor-binding assays, enzymatic assays, immunocytochemical, immunohistochemical assays and cell based assays utilizing fluorometric or time-resolved fluorometric determination of specific luminescence. Chelates of the present invention provide means to obtain improved bioaffinity based binding assays related to e.g. assay sensitivity, kinetics and background.

### Luminescent lanthanide chelate

One aspect of the present invention relates to a luminescent lanthanide chelate of the formula (I) 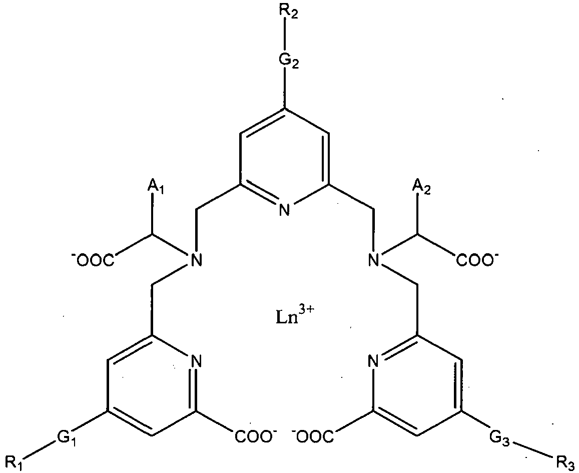 wherein
each of G₁, G₂ and G₃ is independently selected from i) a conjugating group and ii) a single bond, provided that at least one of G₁, G₂ and G₃ is independently a conjugating group, in particular two or all three of G₁, G₂ and G₃ are independently a conjugating group;
each of R₁, R₂, and R₃ is independently selected from i) a reactive group Z, ii) a hydrophilic group, and iii) hydrogen, or the respective group, R₁, R₂, and R₃, is absent;
each of A₁ and A₂ is independently selected from i) a reactive group Z, ii) a hydrophilic group, and iii) hydrogen or C₁₋₆-alkyl;
wherein at least one of R₁, R₂, R₃, A₁ and A₂ is a reactive group Z, and
Ln³⁺ is a lanthanide ion.

At least one of the groups G₁, G₂ and G₃ is a conjugating group. In such instances, such conjugating groups, G₁, G₂, and/or G₃, are groups conjugated with the pyridines. These groups may be substituted with various groups to improve water solubility and/or to shift excitation wavelength and/or to enhance molar excitation coefficient. Any group of G₁, G₂ and G₃ not being a conjugating group simply represents a single bond between the pyridine and R₁, R₂ or R₃, respectively.

In one interesting embodiment at least two of G₁, G₂ and G₃ are independently a conjugating group.

In the currently most interesting embodiments, each G₁, G₂ and G₃ is independently a conjugating group.

In some embodiments, the conjugating group consists of one, two or three moieties, each moiety being selected from ethenylene (-CH=CH-), ethynediyl (-C≡C-), carbonyl (-C(=O)-), and biradicals of (hetero)aromatic ring or ring systems (-Het/Ar-), e.g. phenylene, biphenylene, naphthylene, pyridylene, pyrazinylene, pyrimidinylene, pyridazinylene, furylene, thienylene, pyrrolylene, imidazolylene, pyrazolylene, thiazolylene, isothiazolylene, oxazolylene, isoxazolylene, fyrazanylene, 1,2,4-triazol-3,5-ylene, and oxadiazolylene.

The groups are arranged so as to be conjugated with each other and are attached to the respective pyridine in such a way that the conjugating group is conjugated with the pyridine.

Each of the biradicals of (hetero)aromatic ring or ring systems (-Het/Ar-) may be unsubstituted, mono-R₄-substituted, di-R₄,R₅-substituted, tri-R₄,R₅,R₆-substituted, tetra-R₄,R₅,R₆R₇-substituted, or penta-R₄,R₅,R₆,R₇,R₈-substituted, wherein each of such possible substituents R₄, R₅, R₆, R₇, and R₈ independently are selected from C₁₋₁₂-alkyl, -(CH₂)₀₋₆COOH, -(CH₂)₀₋₆COO⁻, -(CH₂)₀₋₆SO₃H, -(CH₂)₀₋₆SO₃⁻,-NHC(=O)R₁₀, -NCH₃C(=O)R₁₀, -C(=O)NHR₁₀, -C(=O)NCH₃R₁₀, -NHC(=O)NHR₁₀,-NHC(=S)NHR₁₀, -C(=O)R₁₀, -F, -Cl, -Br, -I, hydroxyl (-OH), mercapto (-SH), -OR₉,-SR₉, and a hydrophilic group, and wherein R₉ is selected from -CF₃, -C₁₋₁₂-alkyl,-(CH₂)₁₋₆COOH, -(CH₂)₁₋₆COO⁻, -(CH₂)₁₋₆SO₃H, -(CH₂)₁₋₆SO₃⁻, NHC(=O)R₁₀, -NCH₃C(=O)R₁₀, -C(=O)NHR₁₀, -C(=O)NCH₃R₁₀, -NHC(=O)NHR₁₀,-NHC(=S)NHR₁₀, -C(=O)R₁₀ and a hydrophilic group, wherein R₁₀ is selected from C₁₋₁₂-alkyl, -(CH₂)₁₋₆COOH, -(CH₂)₁₋₆COO⁻, -(CH₂)₁₋₆SO₃H, -(CH₂)₁₋₆SO₃⁻ and a hydrophilic group.

With different R groups the excitation wavelength can be shifted to longer wavelength, which together with high molar absorptivity (ε) means that the labels could be excited by cheap and small LEDs instead of expensive devices presently in use.

In one interesting embodiment, two of G₁, G₂ and G₃, in particular all three, are independently selected from phenylethynyl (C₆H₅-C≡C-), phenyl, thienyl and furyl, such as phenylethynyl, thienyl and furyl, in particular from phenylethynyl, which each may be substituted (cf. as described above for the biradicals of (hetero)aromatic ring or ring systems).

Each of R₁, R₂, and R₃ is independently selected from i) a reactive group Z, ii) a hydrophilic group, and iii) hydrogen. In the event that the biradicals of (hetero)aromatic ring or ring systems constituting G₁, G₂ and G₃, or a part of G₁, G₂ and G₃, is fully substituted, the respective group, R₁, R₂, and R₃, is absent.

Each of A₁ and A₂ is independently selected from i) a reactive group Z, ii) a hydrophilic group, and iii) hydrogen or C₁₋₆-alkyl, in particular from i) a reactive group Z, ii) a hydrophilic group, and iii) hydrogen.

At least one reactive group is required in the chelate molecule. Typically, the number of Z groups in the chelate molecule is 1, 2, or 3, in particular 1 or 2, more preferably just 1.

The reactive group Z is facilitating the labelling of a biospecific binding reactant, or is facilitating the formation of a covalent bond to a solid support material. In case the chelate has a polymerizing group as reactive group, then the chelate may be introduced in the solid support, e.g. a particle, simultaneously with the preparation of the particles.

Examples of the reactive group are those selected from azido (-N₃), alkynyl (-C≡CH), alkylene (-CH=CH₂), amino (-NH₂), aminooxy (-O-NH₂), carboxyl (-COOH), aldehyde (-CHO), mercapto (-SH), maleimido groups or activated derivatives thereof, including isocyanato (-NCO), isothiocyanato (-NCS), diazonium (-N⁺N), bromoacetamido, iodoacetamido, reactive esters, pyridyl-2-dithio, and 6-substituted 4-chloro-1,3,5-triazin-2-ylamino. In two examples, the reactive group comprises an isothiocyanato (-NCS) group.

The substituents in 6-substituted 4-chloro-1,3,5-triazin-2-ylamino can be selected from the group consisting hydrogen, halogen, alkoxy, aryloxy, amino, alkyl with one to six carbon atoms, substituted amino or thioethers, and preferable selected from the group consisting of chloro, fluoro, ethoxy, 2-methoxyethoxy, 2-cyanoethoxy, 2,2,2-trifluoroethoxy, thiophenoxy or ethoxycarbonyl-thiomethoxy. The substituted amino or thioether is preferable mono- or disubstituted each substituent being preferable independently selected from the group consisting of an alkyl or alkoxy with one to six carbon atoms, phenyl, carbonyl or carboxyl.

It follows that upon reaction with a biospecific binding reactant (see further below), the reactive group Z establishes a link to said biospecific binding reactant, e.g. of one of the following types: a thiourea (-NH-C(=S)-NH-), an aminoacetamide (-NH-CO-CH₂-NH-), an amide (-NH-CO-, -CO-NH-, -NCH₃-CO- and -CO-NCH₃-), and aliphatic thioether (-S-), a disulfide (-S-S-), a 6-substituted-1,3,5-triazine-2,4-diamine, a 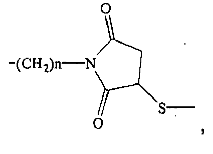 wherein n = 1-6; and a triazole (e.g. formed by the so-called "click" chemistry).

When present, any hydrophilic groups are present in order to, e.g., improve water solubility of the chelate.

Examples of hydrophilic groups are mono- and oligosaccharides, such as monosaccharides and disaccharides, oligoalkylene glycols (e.g. those having 1-20 repeating units) such as oligoethylene glycol and oligopropylene glycol, etc.

In one embodiment, the hydrophilic group is selected from monosaccharides, disaccharides, -(CH₂)₁₋₃-O-(CH₂CH₂O)₀₋₅-H, -(CH₂)₁₋₃-O-(CH₂CH₂O)₀₋₅-C₁₋₄-alkyl,-O-(CH₂CH₂O)₁₋₆-H, and -O-(CH₂CH₂O)₁₋₆-C₁₋₄-alkyl, in particular monosaccharides.

In the present context, the term "monosaccharide" is intended to mean C₅-C₇ carbohydrates being either in the acyclic or in cyclic form. Examples of monosaccharides are C₆ carbohydrates, e.g. those selected from 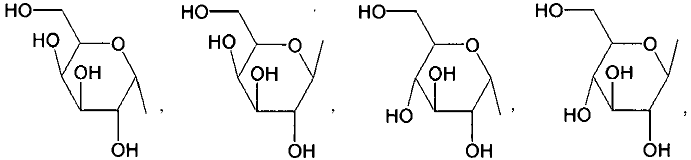 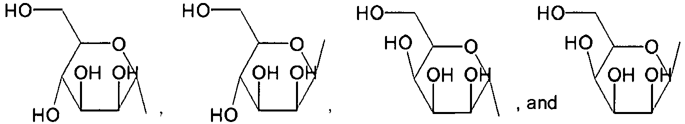 or

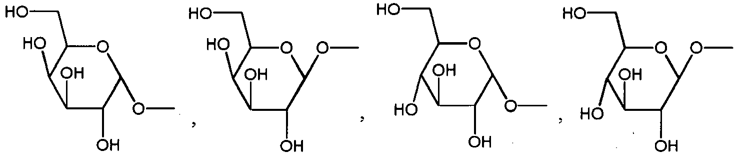

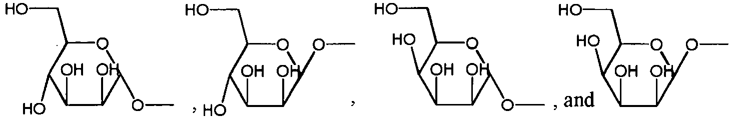

In the present context, the term "disaccharide" is intended to mean two monosaccharides (cf. above) linked together, preferably via glycosidic bonds.

It should be understood that when any of R₁, R₂, R₃, A₁, and A₂ designates a reactive group Z, the group Z may include a spacer, i.e. a distance-making biradical, so as - if necessary or desirable - to position the reactive group Z in a position accessible for reaction with the biospecific binding reactant. Similarly, when any of R₁, R₂, R₃, A₁, and A₂ designates a hydrophilic group, the hydrophilic group may include a spacer. In both instances, the spacer may be readily introduced in the course of the synthesis of the ligand or the chelate. As an example which can easily be generalized (and with reference to Example 3), the reaction between compound 4 and the glycine ethyl ester, it would be possible to use another α-amino acid with a functional side chain (e.g. (protected) glutamic or aspartic acid or protected ornitine or lysine) so as to provide a part of a spacer for establishing a reactive group or a hydrophilic group having included a spacer. An example is given in Scheme 9.

The term "spacer" is intended to mean a distance-making group between, e.g., a conjugating group or a pyridine moiety of the core structure and, e.g. the reactive group Z or a hydrophilic group. The spacer typically has a length of 1-20 bonds between the attachment point and reactive group (or hydrophilic group), such as 3-15 bonds, or 5-12 bonds. The said spacer is formed of one to five moieties, each moiety selected from the group consisting of phenylene, alkylene containing 1-10 carbon atoms, an ethynediyl (-C≡C-), an ether (-O-), a thioether (-S-), a disulfide (-S-S-), an amide (-C(=O)-NH-, -NH-C(=O)-, -C(=O)-NCH₃- and -NCH₃-C(=O)-), a thiourea (-NH-C(=S)-NH-) and a triazole. An example of such a spacer having 11 bonds is illustrated in Scheme 9.

In one embodiment, the hydrophilic group (if present) comprises a spacer which is a distance-making radical, wherein the spacer is as defined above.

In another embodiment, the reactive group Z comprises a spacer which is a distance-making radical, wherein the spacer is as defined above.

In some embodiments, at least one of -A₁ and -A₂ is -H, in particular both of -A₁ and -A₂ are -H.

In other embodiments, at least one of -A₁ and -A₂ is not -H, in particular one of A₁ and A₂ is a reactive group (Z) connected via a spacer, for example -(CH₂)₂-CO-NH-(CH₂)₂-C₆H₄-NCS.

In still another embodiment, at least one of -A₁ and -A₂ is not -H, in particular one of A₁ and A₂ is a hydrophilic group, optionally connected via a spacer.

The term "lanthanide ion" or "Ln³⁺" is intended to mean a trivalent ion of the lanthanide series of the Periodic Table of Elements, e.g. europium(III), terbium(III), samarium(III) and dysprosium(III), i.e. Eu³⁺, Tb³⁺, Sm³⁺ or Dy³⁺. In many embodiments, europium(III) (Eu³⁺) and terbium(III) (Tb³⁺) are preferred.

It should be understood that the basic structure lanthanide chelate of the formula (I) (as well as the lanthanide chelating ligand of the formula (II); see further below) comprises at least four negative charges, and even more negative charges if some substituents are of the type -COO⁻ or -SO₃⁻). Hence, it should be understood that the lanthanide chelate and the lanthanide chelating ligand, respectively, in addition to what is illustrated in formula (I) and formula (II) are further associated with one or more cations as counter ions. Examples of such counter ions are Na⁺, Ca²⁺, K⁺. Particularly preferred are Na⁺ and K⁺. Preferably, the counter ions are those from Groups IA and IIA of the periodic table of elements.

Moreover, the invention provides highly luminescent labels for all lanthanides which provides multi-label possibilities

### Lanthanide chelating ligand

Hence, another aspect of the present invention relates to a lanthanide chelating ligand of the formula (II), 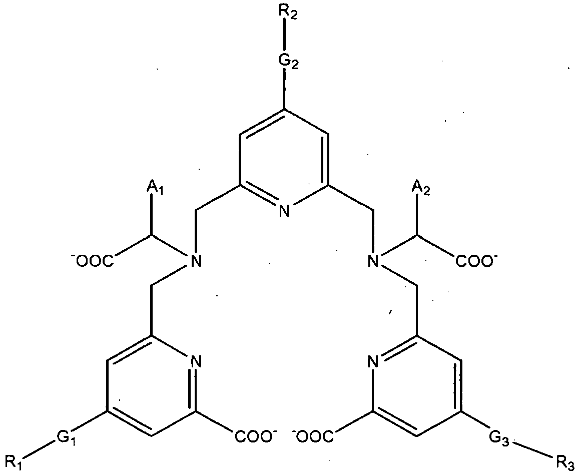 and wherein each of G₁, G₂, G₃, R₁, R₂, R₃, A₁ and A₂ represents the groups G₁, G₂, G₃, R₁, R₂, R₃, A₁ and A₂ as defined for formula (I).

If the ligand is to be used in peptide or oligonucleotide synthesis as a means to prepare a labeled peptide or a labeled oligonucleotide as described in e.g. US 2005/0181393, it is preferable to use one functional group for attaching the ligand to the peptide's or oligonucleotide's back-bone during the synthesis in R₁, R₂, R₃, A₁ or A₂ as described in US 2005/0181393.

### Preferred embodiments

In some preferred embodiments, the chelate molecule comprises a single reactive group Z, in particular as the substituent R₂, e.g. -NCS.

In further embodiments, -G₂-R₂ is a -ethynylene-phenylene-R₂ wherein R₂ is a reactive group, e.g. -NCS.

In some preferred embodiments, the lanthanide chelate of formula (I) is one wherein each of G₁, G₂ and G₃ is phenylethynyl, each of -A₁ and -A₂ is -H, each of R₁ and R₃ is a hydrophilic group, such as a monosaccharide, e.g. 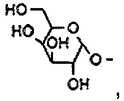 and R₂ is a reactive group, e.g. -NCS.

In another embodiment, the lanthanide chelate of formula (I) is one wherein each of G₁ and G₃ are 3,5-bis(carboxymethoxy)phenylethynyl and G₂ is phenylethynyl, each of -A₁ and -A₂ is -H, each of R₁ and R₃ is -H, and R₂ is a reactive group, e.g. -NCS.

In still another embodiment, the lanthanide chelate of formula (I) is one wherein each of G₁, G₂ and G₃ is phenylethynyl, one of -A₁ and -A₂ is -H, and the other of -A₁ and -A₂ is a reactive group including a spacer, e.g. -(CH₂)₂-CO-NH-(CH₂)₂-C₆H₄-NCS, and each of

R₁, R₂ and R₃ is a hydrophilic group, such as a monosaccharide, e.g.

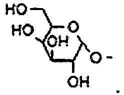

In still another embodiment, the lanthanide chelate of formula (I) is one wherein each of G₁ and G₃ are 4-(carboxymethylthio)phenylethynyl and G₂ is phenylethynyl, each of -A₁ and -A₂ is -H, each of R₁ and R₃ is -H, and R₂ is a reactive group, e.g. -NCS.

In still another embodiment, the lanthanide chelate of formula (I) is one wherein each of G₁ and G₃ are 2,4-bis(carboxymethylthio)phenylethynyl and G₂ is phenylethynyl, each of -A₁ and -A₂ is -H, each of R₁ and R₃ is -H, and R₂ is a reactive group, e.g. -NCS.

In another embodiment, the lanthanide chelate of formula (I) is one wherein each of G₁ and G₃ are 2,4,6-tris(carboxymethoxy)phenylethynyl and G₂ is phenylethynyl, each of -A₁ and -A₂ is -H, each of R₁ and R₃ is -H, and R₂ is a reactive group, e.g. -NCS.

In another embodiment, the lanthanide chelate of formula (I) is one wherein each of G₁ and G₃ are furan-2-yl and G₂ is phenylethynyl, each of -A₁ and -A₂ is -H, each of R₁ and R₃ is -H, and R₂ is a reactive group, e.g. -NCS.

In another embodiment, the lanthanide chelate of formula (I) is one wherein each of G₁ and G₃ are thien-2-yl and G₂ is phenylethynyl, each of -A₁ and -A₂ is -H, each of R₁ and R₃ is -H, and R₂ is a reactive group, e.g. -NCS.

In another embodiment, the lanthanide chelate of formula (I) is one wherein each of R₁-G₁ and R₃-G₃ are (2,4-di(NaOOC-CH₂-O-)phenyl)ethynyl, G₂ is (2-(NaOOC-CH₂-O-)phenyl)ethynyl, each of -A₁ and -A₂ is -H, and R₂ is a reactive group, e.g. -NCS.

In these embodiments, the lanthanide ion (Ln³⁺) is preferably selected from Sm³⁺ and Eu³⁺, in particular Eu³⁺.

The corresponding lanthanide chelating ligands of the formula (II) are equally interesting. The lanthanide chelating ligand is especially suitable for use in peptide synthesis, oligonucleotide synthesis and for solid supports.

### A detectable molecule

Still another aspect of the present invention relates to a detectable molecule comprising a biospecific binding reactant conjugated to a luminescent lanthanide chelate as defined hereinabove. Conjugation is typically obtained by means of a reactive group of said chelate.

The biospecific binding reactant should be capable of specifically binding an analyte of interest for the purpose of quantitative or qualitative analysis of said analyte in a sample.

Examples of biospecific binding reactants are those selected from an antibody, an antigen, a receptor ligand, a specific binding protein, a DNA probe, a RNA probe, an oligopeptide, an oligonucleotide, a modified oligonucleotide (e.g. an LNA modified oligonucleotide), a modified polynucleotide (e.g. an LNA modified polynucleotide), a protein, an oligosaccaride, a polysaccharide, a phospholipid, a PNA, a steroid, a hapten, a drug, a receptor binding ligand, and lectine.

In a preferred embodiment, the biospecific binding reactant is selected from antibodies, e.g. Troponin I antibodies (anti-Tnl).

### A method for carrying out a biospecific binding assay

A still further aspect of the invention relates to a method of carrying out a biospecific binding assay, wherein the method comprises the steps of:
a) forming a biocomplex between an analyte and a biospecific binding reactant labelled by lanthanide chelate as defined herein;
b) exciting said biocomplex with radiation having an excitation wavelength, thereby forming an excited biocomplex;
and c) detecting emission radiation emitted from said excited biocomplex.

The method follows the conventional assay steps as will be evident for the skilled person.

This being said, a further aspect of the invention relates to the use of a detectable molecule as defined above in a specific bioaffinity based binding assay utilizing time-resolved fluorometric determination of a specific luminescence. In one embodiment, the specific bioaffinity based binding assay is a heterogeneous immunoassay, a homogenous immunoassay, a DNA hybridization assay, a receptor binding assay, an immunocytochemical or an immunohistochemical assay.

### A solid support

Still another aspect of the invention relates to a solid support material conjugated with a luminescent lanthanide chelate as defined hereinabove. The luminescent lanthanide chelate is typically immobilized to the solid support material either covalently or non-covalently.

In some interesting embodiments, the solid support material is selected from a nanoparticle, a microparticle, a slide, a plate, and a solid phase synthesis resin.

The novel nine-dentate lanthanide chelates ligands and the corresponding luminescent lanthanide chelates and labeled biospecific binding reactant are based on an open chain, i.e. acyclic, ligand structure with three chromophores which provides surprisingly efficiently excitation of the chelated lanthanide ion. At the same time, all important features of the luminescent lanthanide chelate and labeled biospecific binding reactant can be retained without any additional formation of aggregates and purification problems.

The chelates of the present invention aim to combine several important features in a single label such as:
(a) high absorptivity, preferable over 50,000 cm⁻¹ at suitable wavelength, preferable over 300 nm,
(b) three separate UV absorbing parts (chromophores, triplet sensitizer) in the same ligand structure to allow the high absorptivity of the reactant,
(c) effective energy transfer from the chromophores to the lanthanide ion,
(d) a nine dentate chelating completely protection of the chelated ion against and the well-known luminescence quenching of water molecules,
(e) a strongly chelating part to create i) thermodynamic stability required for storing the labeled reactants for extended period of time, and ii) high kinetic stability to allow the labeled reactants to be used in conditions where competing metal ions or chelating agents may be present,
(f) functional groups to allow high water solubility, to prevent formation of aggregated during labeling of biomolecules and unspecific binding of labeled biomolecules, and to allow the UV excitation over 300 nm and high absorptivity,
(g) optimal amount of CH₂ groups in the close vicinity of the emittive lanthanide ion. Thus, the non-radiative quenching of the ion luminescence through C-H bond vibrational energy manifolds is less significant to offer enhanced luminescence, and
(h) with europium chelates the ligand field with the invented chelate emphasizes the emission line intensity at ca 615 nm over the other emissions lines to offer enhanced luminescence.

### EXAMPLES

The following non-limiting examples are aimed to further demonstrate the invention. The structures and synthetic routes employed are presented in Schemes 1-19. One key synthesis step is to use so called Sonogashira reaction to prepare the conjugated triple bond structures. It is obvious for any person skilled in the art to use known synthetic Stille and Suzuki methods described e.g. in Bioconjugate Chem., 20 (2009) 404 and references situated herein to prepare the e.g. phenyl, thienyl or furyl based lanthanide chelates according to the present invention.

¹H-NMR spectra were recorded with Bruker AVANCE DRX 500 MHz. Tetramethyl silane was used as internal reference. Mass spectra were recorded either on Applied Biosystems QSTAR XL ESI-TOF instrument or PerSeptive Biosystems Voyager DE-PRO MALDI-TOF instrument using α-cyano-4-cinnamic acid matrix. UV-Vis spectra were recorded on Pharmacia Ultrospec 3300 pro. Fluorescence efficiencies were determined with Perkin-Elmer Wallac Victor platefluorometer. Luminescence decay times, excitation and emission spectra as well as fluorescence lifetimes were measured using Varian Cary Eclipse spectrofluorometer. Column chromatography was performed with columns packed with silica gel 60 (Merck).

### Example 1. Synthesis of 2,6-bis(hydroxymethyl)-4-iodopyridine (2)

Diethyl 4-iodo-2,6-pyridinedicarboxylate (1) (5.57 g, 17.3 mmol) was suspended in ethanol (130 ml). Sodium borohydride (2.95 g, 78 mmol) was added in small portions to the stirred solution during 15 min. The mixture was refluxed for 1h 30 min and allowed then to cool down to RT. The reaction mixture was evaporated to dryness and the residue was suspended in saturated aqueous sodium hydrogen carbonate (35 ml). The suspension was rapidly boiled up, allowed to cool and evaporated to dryness. The residue was suspended in mixture of DMF and dichloromethane (1:1, 65 ml), filtrated through Celite pad and the filtrate was evaporated to dryness. The product was crystallized from water and dried in vacuum desiccator over silica gel. Yield 3.37 g (60%). ¹HNMR (CDCl₃, δ ppm): 7.70 (2H, s), 5.46 (2H, s), 4.48 (4H, s).

Compound 1 was prepared as described in Takalo H. and Kankare J., Acta Chemica Scandinavica B 41, 1987, 219-221.

### Example 2. Synthesis of 2,6-bis(bromomethyl)-4-iodopyridine(3)

Phosphorus tribromide (2.45 ml, 26 mmol) was added drop-wise to anhydrous DMF (19.5 ml) at 0 °C resulting in a semisolid mixture. 2,6-bis(hydroxymethyl)-4-iodopyridine (2) (3.36 g, 12.7 mmol) was added to the mixture in small portions. The solid mixture solubilizes during the addition. The mixture was allowed to warm up to RT and stirred 4 h at RT. The reaction mixture was poured to 100 ml of aqueous sodium hydrogen carbonate (5%). The precipitated product was collected by filtration, washed with water and dried in vacuum desiccator over silica gel. Yield 4.51 g (91%). ¹HNMR (CDCl₃, δ ppm): 7.76 (2H, s), 4.45 (4H, s)

### Example 3. Synthesis of compound ethyl-2-((4-bromo-6-(carboxyethyl)-pyridine-2-yl)methylenenitrilo)acetate (5)

4-bromo-6-bromomethyl-2-carboxyethylpyridine (**4**) (2.99 g, 9.3 mmol) and glycine ethyl ester hydrochloride (6.52 g, 46.7 mmol) were dissolved in mixture of anhydrous acetonitrile (125 ml) and di-isopropylethylamine (16.5 ml). Mixture was stirred overnight at RT. Reaction mixture was evaporated to dryness and the product was purified with column chromatography using silica as a stationary phase and dichloromethane:methanol (97:3) as an eluent. Yield 2.78 g (86%). ¹HNMR: (CDCl₃, δ ppm): 8.14 (1H, d, J=1.65Hz), 7.85 (1H, d, J=1.60Hz), 4.47 (2H, q, J=7.12Hz), 4.20 (2H, q, J=7.13Hz), 4.05 (2H, s), 3.47 (2H, s), 2.26 (1H, s), 1.43 (3H, t, J=7.13Hz), 1.28 (3H, t, J=7.13Hz).

Compound 4 was prepared as described in Takalo et al., Helv. Chim. Acta, 1996, 7.9, 789-802.

### Example 4. Synthesis of diethyl 6,6'-((((4-iodopyridine-2,6-diyl)bis(methylene))bis((2-ethoxy-2-oxoethyl)azanediyl))bis(methylene))bis(4-bromopicolinate) (6)

Compound 3 (0.94 g, 2.4 mmol) and compound 5 (1.66 g, 4.8 mmol) were dissolved in anhydrous acetonitrile (80 ml). Anhydrous potassium carbonate (1.67 g, 12 mmol) was added and the mixture was stirred 4 hours at 55-60 °C. Reaction mixture was allowed to cool down to RT and filtrated through Celite pad. The filtrate was evaporated to dryness and the product was purified with column chromatography using silica as a stationary phase and dichloromethane:methanol (95:5) as an eluent. Yield 1.57 g (71%). ¹HNMR: (CDCl₃, δ ppm): 8.12 (2H, d, J=1.60Hz), 8.07 (2H, d, J=1.40Hz), 7.76 (2H, s), 4.46 (4H, q, J=7.12Hz), 4.18 (4H, q, J=7.13), 4.08 (4H, s), 3.92 (4H, s), 3.49 (4H, s), 1.42 (6H, t, J=7.13), 1.28 (6H, t, J=7.13). MS(ESI-TOF) calculated for C₃₃H₃₈Br₂IN₅O₈ [M+H]⁺: 918.02, found: 917.92

**Example 5.** Synthesis of diethyl 6,6'-((((4-((4-aminophenyl)ethynyl)pyridine-2,6-diyl)bis(methylene))bis((2-ethoxy-2-oxoethyl)azanediyl))bis(methylene))bis(4-bromopicolinate) (7)

Compound 6 (412 mg, 0.45mmol) was dissolved in anhydrous dichloromethane (6 ml). Triethylamine (2 ml), copper (I) iodide (3.2 mg, 0.017 mmol), bis(triphenylphosphine)palladium(II) dichloride (9.5 mg, 0.014 mmol) and 4-ethynylaniline (52.5 mg, 0.45 mmol) were added. The reaction mixture was deaerated with argon and the mixture was stirred 3 h at RT. Reaction mixture was evaporated to dryness and the product was purified with column chromatography using silica as a stationary phase and dichloromethane:methanol (90:10) as an eluent. Yield 193 mg (47%). ¹HNMR: (CDCl₃, δ ppm): 8.11 (2H, d, J=1.35Hz), 7.83 (2H, s), 7.32 (2H, d, J=8.15Hz), 7.18 (2H, s), 6.67 (2H, d, J=8.45Hz) 4.38 (4H, s), 4.30 (4H, q, J=6.74Hz), 4.01 (4H, q, J=7.10Hz), 3.97 (4H, s), 3.39 (4H, s), 1.32 (6H, t, J=6.90Hz), 1.17 (6H, t, J=6.93Hz). MS(ESI-TOF) calculated for C₄₁H₄₄Br₂N₆O₈ [M+H]⁺: 907.17, found: 907.05

### Example 6. Synthesis of diethyl 6,6'-((((4-((4-aminophenyl)ethynyl)pyridine-2,6-diyl)bis(methylene))bis((2-ethoxy-2-oxoethyl)azanediyl))bis(methylene))bis((4-(4-(2,3,4,6-tetra-O-acetyl-α-galactopyranoxy)phenyl)ethynyl)picolinate) (9)

Compound 7 (190 mg, 0.21 mmol) and compound 8 (240 mg, 0.54 mmol) were dissolved in anhydrous tetrahydrofuran (3 ml). Triethylamine (3 ml), copper (I) iodide (2.3 mg, 0.012 mmol), bis(triphenylphosphine)palladium(II) dichloride (4.3 mg, 0.006 mmol) (63.8 mg, 0.54 mmol) were added. The reaction mixture was deaerated with argon and the mixture was stirred overnight at 55-60 °C. Reaction mixture was evaporated to dryness and the product was purified with column chromatography using silica as a stationary phase and dichloromethane:methanol (90:10) as an eluent. Yield 155 mg (44%). MS(ESI-TOF) calculated for C₈₅H₉₀N₆O₂₈ [M+H]⁺: 1643.59, found: 1643.51

### Example 7. Synthesis of 6,6'-((((4-((4-aminophenyl)ethynyl)pyridine-2,6-diyl)bis(methylene))bis((carboxylatomethyl)azanediyl))bis(methylene))bis((4-(4-α-galactopyranoxy)phenyl)ethynyl)picolinate europium (10)

Compound 9 (152 mg, 0.092 mmol) was dissolved in 0.5M KOH in ethanol (7.7 ml) and water was added (3.6 ml). The mixture was stirred at RT for 2 h. The mixture was evaporated to dryness, the residue was dissolved in water (2.5 ml) and pH was adjusted to 6.5 by addition of hydrochloric acid (6M). Europium chloride (34 mg, 0.092 mmol) dissolved in water (920 µl) was added to the reaction mixture. Reaction mixture was stirred at RT for 2 hours and the pH was maintained at 6.3-6.5 by addition of sodium hydrogen carbonate or hydrochloric acid to the mixture. The product precipitated out from the reaction mixture and was isolated by centrifugation. The product was dried overnight in vacuum desiccator over silica gel. Yield 115 mg. MS(ESI-TOF) calculated for C₆₁H₅₄EuN₆O₂₀- [M+2H]⁺: 1345.28, found: 1345.25 UV/VIS: λ_{max,abs} (H₂O) = 331 nm.

### Example 8. Synthesis of 6,6'-((((4-((4-isothiocyanatophenyl)ethynyl)pyridine-2,6-diyl)bis(methylene))bis((carboxylatomethyl)azanediyl))bis(methylene))bis((4-(4-α-galactopyranoxy)phenyl)ethynyl)picolinate europium (11)

Aqueous solution of compound 10 (108 mg, 0.08 mmol) was added to a mixture of thiophosgene (42µl, 0.55mmol), NaHCO₃ (52 mg, 0.61 mmol) and chloroform (1.65 ml). After stirring of 1 h at RT, the aqueous phase was washed with chloroform (3x 1ml). The pH was then adjusted to 6.8-7.2 with 1 M acetic acid and the product was precipitated with acetone (33ml). The product was collected by centrifugation, the precipitate was washed with acetone (3x30 ml) and dried overnight in vacuum desiccator over silica gel. MS(ESI-TOF) calculated for C₆₂H₅₂EuN₆O₂₀S⁻ [M-] 1385.22, found 1385.98. UV/VIS: λ_{max,abs} (H₂O) = 328 nm, ε=86000 mol⁻¹ cm⁻¹

### Example 9. Synthesis of 1-Bromo-3,5-dihydroxybenzene (13)

1-bromo-3,5-dimethoxybenzene (**12**) (1.00 g, 4.60 mmol) was dissolved in dry dichloromethane (40ml) and cooled in an ice bath. Boron tribromide (1.33 ml, 13.82 mmol) was added and the mixture was stirred on 2 h. The mixture was allowed to warm up to room temperature and stirred overnight. Methanol (1.4 ml) was added drop-wise to terminate the reaction, and the mixture was poured into water (50 ml) and stirred at RT for 2 h. Reaction mixture was neutralized with NaHCO₃ and the mixture extracted twice with ethyl acetate (30 ml). Combined organic layers were dried over Na₂SO₄ and evaporated to dryness. Product was purified by column chromatography using silica gel as stationary phase and methanol:dichloromethane (5:95) as eluent. Product was a white solid. Yield: 0.71 g (81%). ¹HNMR (DMSO-d6, δ ppm): 9.68 (2H, s), 6.38 (2H, d, J = 2 Hz), 6.19 (1 H, dd, J = 2 Hz).

### Example 10. Synthesis of di-tert-butyl 2,2'-((5-bromo-1,3-phenylene)bis(oxy))diacetate (14)

Compound 13 (0.49 g, 2.59 mmol) was dissolved in DMF (10 ml, dry). Anhydrous K₂CO₃ (2.15 g, 15.56 mmol) and *tert*-butyl bromoacetate (1.15 ml, 7.78 mmol) were added and the mixture was stirred overnight under argon atmosphere at 50°C. Water (17 ml) was added and the mixture was extracted with ethyl acetate (3x40 ml). Combined organic extracts were dried over NaHCO₃ and evaporated to dryness. Crude product was purified by column chromatography using silica gel as stationary phase and dichloromethane as eluent. Product was a white solid. Yield: 0.95 g (87%) ¹H NMR (CDCl₃, δ ppm): 6.67 (2H, d, J = 2 Hz), 6.42 (1H, dd, J = 2 Hz), 1.49 (18H, s)

### Example 11. Synthesis of di-tert-butyl 2,2'-((5-((trimethylsilyl)ethynyl)-1,3-phenylene)bis(oxy))diacetate (15)

Compound **14** (2.00 g, 4.79 mmol) was dissolved in DMF (4 ml, dry) and the solution was placed in a microwave reaction vial. Diethyl amine (12 ml, dry), Pd(PPh₃)₂Cl₂ (168 mg, 0.24 mmol), CuI (46 mg, 0.24 mmol) and PPh₃ (251 mg, 0.96 mmol) were added and the vial was sealed in an argon atmosphere. Trimethylsilyl acetylene (996 µl, 7.19 mmol) was added through a septum and the mixture was stirred at 120°C for 30 minutes using microwave heating. Reaction mixture was evaporated to dryness, dissolved in dichloromethane and purified by column chromatography using silica gel as stationary phase and ethyl acetate:petroleum ether (10:90) as eluent. Yield: 1.36 g (65%) ¹H NMR (CDCl₃, δ ppm): 6.61 (2H, d, J = 2.35 Hz), 6.49 (1H, dd, J = 2.3 Hz), 4.46 (4H, s), 1.49 (18H, s), 0.23 (9H, m).

### Example 12. Synthesis of di-tert-butyl 2,2'-((5-ethynyl-1,3-phenylene)bis(oxy))diacetate (16)

Compound **15** (1.29 g, 2.98 mmol) was dissolved in dichloromethane (40 ml, dry). Tetrabutyl ammonium fluoride (0.934 g, 3.57 mmol) was added and the mixture was stirred in argon atmosphere at room temperature for 45 min. Mixture was washed with 10% citric acid solution (20 ml), and four times with water (4x40 ml). The organic phase was dried over Na₂SO₄ and evaporated to dryness. Crude product was purified by column chromatography using silica gel as stationary phase and ethyl acetate:petroleum ether (10:90) as eluent. Product was a yellowish solid. Yield: 895 mg (83%). ¹H NMR (CDCl₃, δ ppm): 6.64 (2H, d, J = 2.3 Hz), 6.51 (1H, dd, J = 2.33 Hz), 4.47 (4H, s), 3.02 (1H, s), 1.49 (18H, s).

### Example 13. Synthesis of tetra-tert-butyl 2,2',2",2"'-((((6,6'-((((4-((4-aminophenyl)ethynyl)pyridine-2,6-diyl)bis(methylene))bis((2-ethoxy-2-oxoethyl)azanediyl))bis(methylene))bis(2-(ethoxycarbonyl)pyridine-6,4-diyl))bis(ethyne-2,1-diyl))bis(benzene-5,3,1-triyl))tetrakis(oxy))tetraacetate (17)

Compound **16** (0.167 g, 0.459 mmol) and compound **7** (0.167 g, 0.184 mmol) were dissolved in mixture of anhydrous tetrahydrofurane (4 ml) and triethylamine (2.5 ml). Bis(triphenylphosphine)palladium(II) dichloride (3.8 mg, 0.0054 mmol) and copper (I) iodide (2.3 mg, 0.012 mmol) were added and the mixture was stirred overnight under argon atmosphere at 55-60°C. Reaction mixture was evaporated to dryness and purified by column chromatography using silica gel as stationary phase and methanol:dichloromethane (10:90) as eluent. Yield: 71 mg (27%) MS(ESI-TOF) calculated for C₈₁H₉₄N₆O₂₀ [M+H]⁺: 1471.66, Found: 1471.68

### Example 14. Synthesis of 6,6'-((((4-((4-aminophenyl)ethynyl)pyridine-2,6-diyl)bis(methylene))bis((carboxylatomethyl)azanediyl))bis(methylene))bis(4-((3,5-bis(carboxymethoxy)phenyl)ethynyl)picolinate europium(III) (18)

Compound 17 (68 mg, 0.048 mmol) was dissolved in trifluoroacetic acid (1 ml, 13.5 mmol) and the mixture was stirred at RT for 1.5 h. Reaction mixture was evaporated to dryness without heating. Diethyl ether (2.5ml) was added to the residue and the mixture was stirred for 15 minutes. Product precipitated from the mixture and was isolated by centrifugation. The precipitate was washed twice with diethyl ether and then dried in vacuum desiccator over silica gel. Dried precipitate (64 mg, 0.051 mmol) was dissolved in the mixture of water (1.8 ml) and 0.5M potassium hydroxide solution in ethanol (4 ml). Mixture was stirred in RT for 2 h. Reaction mixture was evaporated to dryness and the residue was dissolved in water (1.7 ml). pH was adjusted to 6.5 by addition of hydrochloric acid (6M). Europium chloride (20 mg, 0.055 mmol) dissolved in water (485 µl) was added to the reaction mixture drop-wise. Reaction mixture was stirred at RT for 2 hours and the pH was maintained at 6.3-6.5 by addition of sodium hydrogen carbonate to the mixture. Excess of europium was precipitated from the mixture by adjusting pH to 8.5-9.0 by addition of sodium hydroxide and the precipitate was removed by centrifugation. The product was precipitated with acetone (25 ml) and isolated by centrifugation. The precipitate was washed with acetone (3x10 ml) and dried overnight in vacuum desiccator over silica gel. Yield: 180 mg. MS(ESI-TOF) calculated for C₅₇H₄₂EuN₆O₂₀⁻ [M+2H]⁺(for free acids without sodium ions): 1285.18, Found: 1285.24

### Example 15. Synthesis of 6,6'-((((4-((4-isothiocyanatophenyl)ethynyl)pyridine-2,6-diyl)bis(methylene))bis((carboxylatomethyl)azanediyl))bis(methylene))bis(4-((3,5-bis(carboxymethoxy)phenyl)ethynyl)picolinate) europium(III) (19)

Aqueous solution of compound **18** (175 mg, 0.143 mmol) was added drop-wise to a mixture of chloroform (2.85 ml), thiophosgene (74 µl, 0.97 mmol) and sodium hydrogen carbonate (91 mg, 1.09 mmol). Reaction mixture was stirred at RT for 45 min. The two phases were separated and the aqueous phase was washed with chloroform (3x6 ml). pH was adjusted to 7 with 1 M acetic acid and the product was precipitated with acetone (57 ml) and isolated by centrifugation. The precipitate was washed with acetone (3x15 ml) and dried overnight in vacuum desiccator over silica gel. Yield: 216 mg. MS(ESI-TOF) calculated for C₅₈H₄₀EuN₆O₂₀S- [M-H]²⁻(for free acids without sodium ions): 662.56, Found: 662.47

### Example 16. Comparison between luminescence of Eu-chelate according to present invention and a conventional 9-dentate α-galactose Eu chelate

Compound **11** (5.3 mg) and a conventional 9-dentate α-galactose Eu chelate (**21**)(5.5 mg) were coupled to taurine (5 mg for each reaction) by dissolving the starting materials in to a mixture of aqueous sodium hydrogen carbonate (2 ml, 50 mM, pH9.8) and DMF (200 µl). The mixtures were incubated overnight at RT. The products (**20** and **22**) were purified by using reversed phase HPLC (RP-18 column). The solvents were A: Triethyl ammonium acetate buffer (20mM, pH7) and B: 50% acetonitrile in triethyl ammonium acetate buffer (20mM, pH7). The gradient was started from 5% of solvent B and the amount of solvent B was linearly raised to 100 % in 25 minutes. The products eluted from the column at 16.7 min (**20**) and at 15.1 min (**22**) time points. Fractions containing the products were collected, pooled and evaporated to dryness.

The conventional 9-dentate α-galactose Eu chelate (**21**) was prepared according to von Lode P. et al., Anal. Chem., 2003, 75, 3193-3201.

The purified products were dissolved in water and analyzed for UV spectrum, Eu-content with DELFIA^{®} against Eu standard material and luminescence signal in water with Victor™ Plate fluorometer. In the luminescence measurements the concentrations of compounds **20** and **22** were adjusted equimolar based on the Eu content. The results are summarized in Table 1.

**Table 1.**

| | **Compound 22 (reference compound)** | **Compound 20** |
|---|---|---|
| Molar absorptivity (ε) based on Eu content | 56,000 | 86,000 |
| Relative luminescence signal (concentration approx. 1 nM) | 195,000 | 633,000 |

These results show that Compound **20** provides surprisingly high luminescence yield compared to the reference compound (more than 300% higher). Earlier published luminescence field (εΦ) for α-gal-9-D Eu (compound **21**) coupled to antibody was 4,787 (von Lode P. et al., Anal. Chem., 2003, 75, 3193-3201). By assuming the same luminescence yield for compound **22** the luminescence yield for compound **20** can be estimated to be: εΦ = 633,000/195,000 x 4,787 = approx. 15,500. That is one of the highest luminescence yield ever seen with a chelate having also a reactive group for labeling a biomolecule.

### Example 17. Labelling of antibody with Compound 19

Labelling of an TnI antibody was performed as described in von Lode P. et al., Anal. Chem., 2003, 75, 3193-3201 by using 90 fold excess of compound **19**. The reactions were carried out overnight at RT. Labelled antibody was separated from the excess of compound **19** on Superdex 200 HR 10/30 gel filtration column (GE healthcare) by using Tris-saline-azide (Tris 50 mM, NaCl 0.9%, pH 7.75) buffer as an eluent. The fractions containing the antibody were pooled and the europium concentration was measured against Eu standard material with Victor™ plate fluorometer. The labelling degree of 1.6 Eu/IgG was obtained.

### Example 18. Troponin I immunoassay

The Tnl antibody labeled with compound **19** was tested in sandwich immunoassay for cardiac troponin I. As a reference compound a TnI antibody labelled with compound **21** (labelling degree 11 Eu/IgG) was used. 10 µl of diluted tracer antibody (5ng/µl) and 20 µl of TnI standard solution were pipetted to a pre-coated assay well (single wells in 96 well plate format, wells coated with streptavidin and a biotinylated capture antibody against Tnl, Innotrac Diagnostics). The reaction mixtures were incubated 20 min at 36°C with shaking. The wells were washed 6 times and dried prior to measurement with Victor™ Plate fluorometer. The results are summarized in Table 2. Both A and B standards were measured in 12 replicates and other standards C-F in 6 replicates.

**Table 2.**

| | **cTnl std** | **A** | **B** | **C** | **D** | **E** | **F** |
|---|---|---|---|---|---|---|---|
| | **(ng/ml)** | **0** | **0,022** | **0,08** | **0,75** | **5,34** | **41,6** |
| | | | | | | | |
| **IgG labeled with compound 19** | **cps-blank** | 197 | 310 | 1030 | 9098 | 64347 | 459222 |
| **reference compound** | **cps-blank** | 195 | 258 | 775 | 6624 | 47913 | 369540 |
| | | | | | | | |
| **IgG labeled with compound 19** | **cv%** | 7,8 | 6,4 | 4,1 | 3,2 | 1,9 | 3,2 |
| **reference compound** | **cv%** | 9,5 | 8,7 | 3,8 | 3,1 | 5,3 | 7,1 |

The results show that compound **19** provides the same signal levels with 1/10 amount of chelate/IgG when compared to the reference compound (**21**). In other words the compound according to present invention provides an order of magnitude more luminescence signal per chelate than the state of the art reference compound.

### Remarks

The chelate is nine-dentate (optimal for lanthanides) and has three separate chromophores which should give 50 % higher luminescence compared to the present nine-dentate chelates in use having two separate chromophores. Compared to other known chelates with two or three chromophores (e.g. NOTA and DOTA based structures) the invented chelate has less CH₂ groups in the close vicinity of the emitting ion. Thus the non-radiative quenching of the ion luminescence through C-H bond vibrational energy manifolds is less significant and thus the luminescence should be more enhanced. Moreover, being acyclic nine-dentate chelate the ligand do not have any sterical hindrance when complexing a lanthanide ion. In that case the distance between the emitting lanthanide ion and pyridine nitrogen is optimal 1) for effective energy transfer from the donor pyridine nitrogen to the lanthanide ion and 2) for high complex stability, and thus offer enhanced luminescence. The chelate synthesis is easier to be performed compared to the other three chromophores containing DOTA based chelates. The three chromophores between each other can be entirely different which means more possible variations and more tailoring possibilities e.g. solubility in different solvents, various excitation wavelengths and energy transfers etc. Cheap and small LEDs can be used to excite the labelled biomolecules. Thus, smaller and cheaper instrumentation for commercial bioassay systems can be used. The ligand field with the invented chelate emphasizes the emission line intensity at approx. 615 nm over the other emissions lines, and thus the observed luminescence should be improved compared to others. Increased chromophoric symmetry is expected to increase luminescence yield.

Surprisingly, the observed luminescence was approx. 300% higher compared to the present chelates in use. The assumption was an approx. 50-70% improvement. This means that it should be possible to improve our assay sensitivities approx. three fold compared to the present sensitivities.

### Example 19. Synthesis of triethyl 2,2',2"-((4-iodo-1,3,5-phenylene)tris(oxy))triacetate (23)

A mixture of 4-Iodo-1,3,5-trihydroxybenzene (3.41 g, 13.5 mmol; Acta Chem. Scand. 1991, 45, 539), dry K₂CO₃ (6.17 g, 44.7 mmol), ethyl bromoacetate (4.96 ml, 44.7 mmol) and dry MeCN (100 ml) was stirred overnight at 55°C. The mixture was filtered, the solid material washed with MeCN and the filtrate was evaporated to dryness. The product was purified by column chromatography using silica gel as stationary phase and MeOH:CH₂Cl₂ (first 0:100, then 20:80) as eluent. Yield: 0.64 g (9%). ¹H NMR (CDCl₃, δ ppm): 6.10 (2H, s), 4.65 (4H, s), 4.55 (4H, s), 4.27 (2H, q, J=7.15 Hz), 4.26 (4H, q, J=7.15 Hz), 1.31 (3H, t, J=7.15 Hz), 1.30 (6H, t, J=7.15 Hz). MS(ESI-TOF) calculated for C₁₈H₂₃IO₉ [M+H]⁺: 511.05, found: 510.93.

### Example 20. Synthesis of triethyl 2,2',2"-((4-(trimethylsilyl)ethynyl-1,3,5-phenylene)tris(oxy))-triacetate (24)

This compound **24** was synthesized from the compound **23** using a method analogous to the synthesis described in the Example 11. The mixture was stirred first at 100°C for 30 minutes, then at 120°C for 20 minutes by using microwave heating. The mixture was extracted with Et₂O (50 ml), washed with H₂O (2 x 20 ml), dried with Na₂SO₄, and the product purified by column chromatography using silica gel as stationary phase and ethyl acetate:petroleum ether (first 20:80, then 30:70) as eluent. Yield: 70%. ¹H NMR (CDCl₃, δ ppm): 6.10 (2H, s), 4.67 (4H, s), 4.55 (2H, s), 4.27 (2H, g, J=7.15 Hz), 4.26 (4H, q, J=7.15 Hz), 1.30 (6H, t, J=7.15 Hz), 1.29 (3H, t, J=7.15 Hz), 0.26 (9H, s). MS(ESI-TOF) calculated for C₂₃H₃₂O₉Si [M+H]⁺: 481.19, found: 481.99.

### Example 21. Synthesis of triethyl 2,2',2"-((4-ethynyl-1,3,5-phenylene)tris(oxy))triacetate (25)

This compound **25** was synthesized from the compound 24 using a method analogous to the synthesis described in the Example 12. After washings with 10% citric acid and H₂O, the product (100%) was used for the next step without further purifications. ¹H NMR (CDCl₃, δ ppm): 6.06 (2H,s), 4.69 (4H, s), 4.55 (2H, s), 4.27 (2H, q, J=7.15 Hz), 4.26 (4H, q, J=7.15 Hz), 3.50 (1H, s), 1.30 (3H, t, J=7.15 Hz), 1.29 (6H, t, J=7.15 Hz). MS(ESI-TOF) calculated for C₂₀H₂₄O₉ [M+H]⁺: 409.15, found: 409.20.

### Example 22. Synthesis of diethyl 2,2'-(1,3-phenylenebis(sulfanediyl))diacetate (26)

A mixture of benzene-1,3-dithiol (0.76 g, 4 mmol), dry K₂CO₃ (2.21 g, 16 mmol), ethyl bromoacetate (0.93 ml, 8.4 mmol) and dry MeCN (20 ml) was stirred overnight at 55°C and under agron. The mixture was filtered, the solid material washed with MeCN and the filtrate was evaporated to dryness. The product was purified by column chromatography using silica gel as stationary phase and CH₂Cl₂ as eluent. Yield: 0.96 g (76%). ¹H NMR (CDCl₃, δ ppm): 7.45-7.43 (1H, m), 7.25-7.20 (3H, m), 4.18 (4H, q, J=7.15 Hz), 3.64 (4H, s), 1.23 (6H, t, J=7.15 Hz). MS(ESI-TOF) calculated for C₁₄H₁₈O₄S₂ [M+H]⁺: 315.07, found: 315.98.

### Example 23. Synthesis of diethyl 2,2'-(4-bromo-1,3-phenylenebis(sulfanediyl))-diacetate (27)

A mixture of the compound **26** (0.64 g, 2.04 mmol) and N-bromosuccinimide (0.47 g, 2.66 mmol) in CH₂Cl₂ (10 ml) was stirred for four days at room temperature. After evaporation to dryness, the product was purified by column chromatography using silica gel as stationary phase and petroleum ether:CH₂Cl₂ (20:80) as eluent. Yield: 0.50 g (63%). ¹H NMR (CDCl₃, δ ppm): 7.45 (1H, d, J=8.32 Hz), 7.42 (1H, d, J=2.18 Hz), 7.09 (1H, dd, J=8.32 and 2.18 Hz), 4.20 (2H, q, J=7.15 Hz), 4.18 (2H, q, J=7.15 Hz), 3.70 (2H, s), 3.63 (2H, s), 1.27 (3H, t, J=7.15 Hz), 1.24 (3H, t, J=7.15 Hz). MS(ESI-TOF) calculated for C₁₄H₁₇BrO₄S₂ [M+H]⁺: 394.98 and 391.99, found: 394.95 and 391.82.

### Example 24. Synthesis of diethyl 2,2'-(4-(trimethylsilyl)ethynyl-1,3-phenylenebis(sulfanediyl))-diacetate (28)

This compound **28** was synthesized from the compound **27** using a method analogous to the synthesis described in the Example 11. The mixture was stirred at 100°C for 30 minutes by using microwave heating. After evaporation to dryness, the product was purified by column chromatography using silica gel as stationary phase and ethyl acetate:petroleum ether (first 10:90, then 20:80) as eluent. Yield: 82%. ¹H NMR (CDCl₃, δ ppm): 7.36 (1H, d, J=1.75 Hz), 7.34 (1H, d, J=8.08 Hz), 7.12 (1H, dd, J=8.08 and 1.75 Hz), 4.19 (2H, q, J=7.13 Hz), 4.18 (2H, q, J=7.13 Hz), 3.73 (2H, s), 3.67 (2H, s), 1.26 (3H, t, J=7.13 Hz), 1.24 (3H, t, J=7.13 Hz), 0.27 (9H, s). MS(ESI-TOF) calculated for C₁₉H₂₆O₄S₂Si [M+H]⁺: 411.11, found: 412.02.

### Example 25. Synthesis of diethyl 2,2'-(4-ethynyl-1,3-phenylenebis(sulfanediyl))diacetate (29)

This compound **29** was synthesized from the compound **28** using a method analogous to the synthesis described in the Example 12. After washings with 10% citric acid and H₂O, the product was purified by column chromatography using silica gel as stationary phase and ethyl acetate:petroleum ether (first 10:90, then 15:85, and finally 20:80 ) as eluent. Yield: 41%. ¹H NMR (CDCl₃, δ ppm): 7.40 (1H, d, J=1.88 Hz), 7.39 (1H, d, J=8.08 Hz), 7.15 (1 H, dd, J=8.08 and 1.88 Hz), 4.19 (2H, q, J=7.15 Hz), 4.18 (2H, q, J=7.15 Hz); 3.73 (2H, s), 3.68 (2H, s), 3.47 (1H, s), 1.25 (6H, t, J=7.15 Hz). MS(ESI-TOF) calculated for C₁₆H₁₈O₄S₂ [M+H]⁺: 339.07, found: 339.83.

### Example 26. Synthesis of ethyl 2-((4-bromophenyl)thio)acetate 30

A mixture of 4-bromothiophenol (0.95 g, 5 mmol), dry K₂CO₃ (1.38 g, 10 mmol), ethyl bromoacetate (0.72 ml, 6.5 mmol) and dry MeCN (20 ml) was stirred three days at room temperature and under agron. The mixture was filtered, the solid material washed with MeCN and the filtrate was evaporated to dryness. The product was purified by column chromatography using silica gel as stationary phase and ethyl acetate:petroleum ether as eluent (first 2:98. then 7:93). Yield: 1.22 g (88%). ¹H NMR (CDCl₃, δ ppm): 7.42 (2H, d, J=8.58 Hz), 7.28 (2H, d, J=8.58 Hz), 4.17 (2H, q, J=7.15 Hz), 3.61 (2H, s), 1.23 (3H, t, J=7.15 Hz). MS(ESI-TOF) calculated for C₁₀H₁₁BrO₂S [M+H]⁺: 276.97 and 274.98, found: 276.66 and 274.63.

### Example 27. Synthesis of ethyl 2-((4-((trimethylsilyl)ethynyl)phenyl)thio)acetate 31

This compound **31** was synthesized from the compound **30** using a method analogous to the synthesis described in the Example 11. The mixture was stirred at 120°C for 40 minutes by using microwave heating. After evaporation to dryness, the product was purified by column chromatography using silica gel as stationary phase and ethyl acetate:petroleum ether (10:90) as eluent. Yield: 88%. ¹H NMR (CDCl₃, δ ppm): 7.38 (2H, d, J=8.38 Hz), 7.30 (2H, d, J=8.38 Hz), 4.17 (2H, q, J=7.15 Hz), 3.64 (2H, s), 1.23 (3H, t, J=7.15 Hz), 0.24 (9H, s), MS (ESI-TOF) calculated for C₁₅H₂₀O₂SSi [M+H]⁺: 294.11, found: 294.11.

### Example 28. Synthesis of ethyl 2-((4-ethynylphenyl)thio)acetate 32

This compound **32** was synthesized from the compound **31** using a method analogous to the synthesis described in the Example 12. After washings with 10% citric acid and H₂O, the product was purified by column chromatography using silica gel as stationary phase and ethyl acetate:petroleum ether (first 5:95, then 10:90) as eluent. Yield: 68%. ¹H NMR (CDCl₃, δ ppm): 7.41 (2H, d, J=8.45 Hz), 7.32 (2H, d, J=8.45 Hz), 4.18 (2H, q, J=7.15 Hz), 3.65 (2H, s), 3.09 (1 H, s), 1.23 (3H, t, J=7.15 Hz). MS(ESI-TOF) calculated for C₁₂H₁₂O₂S [M+H]⁺: 221.07, found: 221.15.

### Example 29. Synthesis of N-(4-ethynylphenyl)-2,2,2-trifluoroacetamide (33)

4-Ethynylaniline (1.38 g, 11.8 mmol) was added in small portions into an ice-cold (CF₃CO)₂O (6.6 ml). After stirring for 10 min in ice-bath, the mixture was stirred for 2.5 hours at room temperature. The mixture was poured into ice-H₂O (100 ml), the product filtered and washed with H₂O. The product (2.29 g, 91%) was used for the next step without further purifications. ¹H NMR (CDCl3): δ (ppm)) 8.08 (1 H, s), 7.55 (2H, d, J=8.80 Hz), 7.51 (2 H, d, J=8.80 Hz), 3.1 (1H, s). MS(ESI-TOF) calculated for C₁₀H₆F₃NO [M+H]⁺: calculated 214.02, found 213.07.

### Example 30. Synthesis of N-(4-((2,6-bis(hydroxymethyl)pyridin-4-yl)ethynyl)phenyl)-2,2,2-trifluoroacetamide (34)

A mixture of the compound **33** (0.55 g, 2.58 mmol) and 6-bromo-2,6-dihydroxymethylpyridine (0.47 g, 2.15 mmol; Acta Chem. Scand. 1988, Ser B, 42, 614) in dry triethylamine (5 ml) and tetrahydrofurane (10 ml) was de-aerated with argon. After addition of bis(triphenylphosphine)-palladium(II) chloride (30 mg, 43 µmol) and Cul (16 mg, 86 µmol), the mixture was stirred for 19 hours at 55°C. After evaporation to dryness, the residue was treated with a cold mixture of CH₂Cl₂ (40 ml) and H₂O (20 ml), filtered and the product (0.56 g, 75%) washed with cold H₂O (10 ml) and CH₂Cl₂ (10 ml). Yield: 0.56 g (75%). ¹H NMR (D6-DMSO, δ ppm): 11.4 (1 H, bs), 7.79 (2H, d, J=8.80 Hz), 7.68 (2H, d, J=8.80 Hz), 7.42 (2H, s), 5.49 (2H, bs), 4.56 (4H, s). MS(ESI-TOF) calculated for C₁₇H₁₃F₃N₂O₃ [M+H]⁺: calculated 351.10, found 351.96.

### Example 31. Synthesis of N-(4-((2,6-bis(bromomethyl)pyridin-4-yl)ethynyl)phenyl)-2,2,2-trifluoroacetamide (35)

PBr₃ (225 µl) was added in a suspension of the compound **34** (0.56 g, 1.6 mmol) in CHCl₃ (65 ml). After stirring for 20 hours at 60°C, the mixture was neutralized with 5% NaHCO₃ (35 ml). The aqueous phase was extracted with CHCl₃ (40 ml) and the combined organic phases were dried with Na₂SO₄, filtered and evaporated to dryness. The product (1.09 g, 93%) was used for the next step without further purifications. ¹H NMR (CDCl₃, δ ppm): 7.93 (1 H, s), 7.63 (2H, d, J=8.68 Hz), 7.59 (2H, J=8.68 Hz), 7.47 (2H, s), 4.53 (4H, s). MS(ESI-TOF) calculated for C₁₇H₁₁Br₂F₃N₂O [M+H]⁺: calculated 474.93, 476.93, 498.92, found 475.38, 477.44, 479.45.

### Example 32. Synthesis of diethyl 6,6'-((((4-((4-trifluoroacetamidophenyl)ethynyl)pyridine-2,6-diyl)bis(methylene))bis((2-ethoxy-2-oxoethyl)azanediyl))bis(methylene))bis(4-bromopicolinate) (36)

This compound **36** was synthesized from the compounds **34** and **5** using a method analogous to the synthesis described in the Example 4. Reaction time at 70 °C was 27 hours. The product was purified by column chromatography using silica gel as stationary phase and triethylamine:ethyl acetate:petroleum ether (1:69:30) as eluent. Yield: 66%. ¹H NMR (CDCl₃, δ ppm): 8.53 (1H, s), 8.14 (2H, d, J=1.80 Hz), 8.11 (2H, d, J=1.80 Hz), 7.68 (2 H, d, J=8.73 Hz), 7.60 (2H, d, J=8.73 Hz), 7.34 (2H, s), 4.44 (4H, q, J=7.08 Hz), 4.18 (4 H, q, J=7.15 Hz); 4.10 (4H, s), 3.98 (4 H, s), 3.50 (4 H, s), 1.40 (6H, t, J=7.08 Hz), 1.28 (6H, t, J=7.15 Hz). MS(ESI-TOF) calculated for C₄₃H₄₃Br₂F₃N₆O₉ [M+H]⁺: calculated 1003.15, 1005.15, 1007.15, found 1003.71, 1005.48, 1007.58.

### Example 33. Synthesis of the compound 37

A mixture of the compound **36** (120 g, 0.148 mmol) and **25** (145 mg, 0.355 mmol) in dry triethylamine (1 ml) and tetrahydrofurane (2 ml) was de-aerated with argon. After addition of bis(triphenylphosphine)palladium(II) chloride (10 mg, 14 µmol) and CuI (6 mg, 28 µmol), the mixture was stirred for 22 hours at 55°C. After evaporation to dryness, the residue was dissolved in CH₂Cl₂ (40 ml), washed with H₂O (3 x 10 ml) and dried with Na₂SO₄. The product was purified by column chromatography using silica gel as stationary phase and triethylamine:ethyl acetate:petroleum ether (1:69:30) as eluent. Yield: 115 mg (47%). ¹H NMR (CDCl₃, δ ppm): 9.12 (1H, s), 8.09 (2H, d, J=1.25 Hz), 8.06 (2H, d, J=1.25 Hz), 7.57 (2 H, s), 7.34 (2H, d, J=8.80 Hz), 7.30 (2H, d, J=8.80 Hz), 5.97 (4H, s), 4.63 (8H, s), 4.60 (4H, s), 4.45 (4H, q, J=7.12 Hz), 4.30 (4H, q, J=7.12 Hz), 4,21 (8H, q, J=7.12 Hz), 4.17 (4H, q, J=7.12), 4.10 (4H, s), 4.02 (4H, s), 3.52 (4H, s), 1.42 (6H, t, J=7.12 Hz), 1.33 (6H, t, J=7.12 Hz), 1.29 (6H, J=7.12 Hz), 1.25 (12H, t, J=7.12 Hz). MS(ESI-TOF) calculated for C₈₃H₈₉F₃N₆O₂₇ [M+H]⁺: calculated 1659.58, found 1659.81.

### Example 34. Synthesis of the compound 38

This compound **38** was synthesized from the compounds **36** and **29** using a method analogous to the synthesis described in the Example 33. The product was purified by column chromatography using silica gel as stationary phase and triethylamine:ethyl acetate:petroleum ether (1:69:30) as eluent. Yield: 85%. ¹H NMR (CDCl₃, δ ppm): 9.22 (1H, s), 8.07 (2H, d. J=1.15 Hz), 8.02 (2H, d, J=1.15 Hz), 7.55 (2H, s), 7.41 (2H, d, J=8.75 Hz), 7.38 (2H, d, J=8.10 Hz), 7.31 (2H, d, J=8.75 Hz), 7.30 (2H, d, J=1.67 Hz), 7.01 (2H, dd, J=8.10 and 1.67 Hz), 4.46 (4H, q, J=7.15 Hz), 4.22 (4H, q, J=7.15 Hz), 4.19 (4H, q, J=7.15 Hz), 4.17 (4H, q, J=7.15 Hz), 4.10 (4H, s), 3.89 (4H, s), 3.74 (4H, s), 3.70 (4H, s), 3,50 (4H, s), 1.42 (6H, t, J=7.15Hz), 1.28 (12H, t, J=7.15 Hz), 1.24 (6H, t, J=7.15 Hz). MS(ESI-TOF) calculated for C₇₅H₇₇F₃N₆O₁₇S₄ [M+H]⁺: calculated 1519.42, found 1519.56.

### Example 35. Synthesis of the compound 39

This compound **39** was synthesized from the compounds **36** and **32** using a method analogous to the synthesis described in the Example 33. The product was purified by column chromatography using silica gel as stationary phase and triethylamine:ethyl acetate:petroleum ether (1:69:30) as eluent. Yield: 81%. ¹H NMR (CDCl₃, δ ppm): 9.29 (1H, s), 8.03 (2H, d. J=1.28 Hz), 8.01 (2H, d, J=1.28 Hz), 7.57 (2H, s), 7.44 (4H, d, J=8.48 Hz), 7.42 (2H, d, J=8.70 Hz), 7.32 (2H, d, J=8.70 Hz), 7.25 (4H, d, J=8.48 Hz), 4.46 (4H, q, J=7.15 Hz), 4.21 (4H, q, J=7.15 Hz), 4.18 (4H, q, J=7.15 Hz), 4.08 (4H, s), 3.95 (4H, s), 3.73 (4H, s), 3.49 (4H, s), 1.42 (6H, t, J=7.15 Hz), 1.28 (6H, t, J=7.15 Hz), 1.27 (6H, t, J=7.15 Hz).

### Example 36. Synthesis of the europium(III) chelate 40

A mixture of the compound **37** (105 mg, 63 µmol) and 0.5M KOH in ethanol (9 ml) was stirred for 30 minutes at room temperature and water was added (2 ml). After stirring for 3 hours at room temperature, EtOH was evaporated, the residue was stirred for 30 minutes at room temperature, and the pH was adjusted to ca. 6.5 with 6M HCl. Europium(III) chloride (23 mg, 63 µmol) in H₂O (0.17 ml) was added within 10 minutes and the pH was maintained at 5-7 with solid NaHCO₃. After stirring for overnight at room temperature, the pH was raised to 8.5 with 1 M NaOH, the precipitate was centrifuged off and the supernatant was extracted with phenol (once with 0.75 g and 3 x 0.5 g). The combined phenol phases were treated with H₂O (1 ml) and Et₂O (20 ml), the aqueous phase was washed with Et₂O (2 x 20 ml), and triturated with acetone. The precipitate was centrifuged and washed with acetone. The product was used for the next step without further purification. Conditions for HPLC run: see example 16. R_{f}(HPLC)=14.5 min. UV/VIS=359 nm.

### Example 37. Synthesis of the europium(III) chelate 41

This compound **41** was synthesized from the compound **38** using a method analogous to the synthesis described in the Example 36. R_{f}(HPLC)=17.3 min. UV/VIS=318, 325 and 350 (sh) nm.

### Example 38. Synthesis of the europium(III) chelate 42

This compound **42** was synthesized from the compound **39** using a method analogous to the synthesis described in the Example 36. R_{f}(HPLC)=21.5 min. UV/VIS=349 nm.

### Example 39. Synthesis of the europium(III) labeling chelate 43

This compound **43** was synthesized from the chelate **40** using a method analogous to the synthesis described in the Example 15. Conditions for HPLC run: see example 16. R_{f}(HPLC)=20.9 min. UV/VIS=325 (sh), 340 and 362 (sh) nm.

### Example 40. Synthesis of the europium(III) labeling chelate 44

This compound **44** was synthesized from the chelate **41** using a method analogous to the synthesis described in the Example 15. Conditions for HPLC run: see example 16. R_{f}(HPLC)=23.3 min. UV/VIS= 305 (sh), 318 and 335 (sh) nm.

### Example 41. Synthesis of the europium(III) labeling chelate 45

This compound **45** was synthesized from the chelate **42** using a method analogous to the synthesis described in the Example 15. Conditions for HPLC run: see example 16. R_{f}(HPLC)=27.9 min. UV/VIS=339 nm.

### Example 42. Synthesis of the europium(III) chelate 46

This compound **46** was synthesized from the chelate **43** using a method analogous to the synthesis described in the Example 16. R_{f}(HPLC)=14.3 min. UV/VIS=349 nm.

### Example 43. Synthesis of the europium(III) chelate 47

This compound **47** was synthesized from the chelate **44** using a method analogous to the synthesis described in the Example 16. R_{f}(HPLC)=17.2 min. UV/VIS=317 and 324 (sh) nm.

### Example 44. Synthesis of the europium(III) chelate 48

This compound **48** was synthesized from the chelate **45** using a method analogous to the synthesis described in the Example 16. R_{f}(HPLC)=20.5 min. UV/VIS=336 nm.

### Example 45. Synthesis of ethyl 2-((4-(furan-2-yl)-6-(carboxyethyl)-pyridine-2-yl)methylenenitrilo)-acetate 49

This compound **49** was synthesized from 4-(furan-2-yl)-6-bromomethyl-2-carboxyethylpyridine (WO2005/021538) using a method analogous to the synthesis described in the Example 3. The product was purified by column chromatography using silica gel as stationary phase and methanol:CH₂CL₂ (5:95) as eluent. ¹H NMR (CDCl₃, δ ppm):8.21 (1H, d, J=1.43 Hz), 7.83 (1H, d, J=1.43 Hz), 7.58 (1H, d, J=1.63 Hz), 6.99 (1H, d, J=3.20 Hz), 6.55 (1H, dd, J= 1.63 and 3.20 Hz), 4.49 (2H, q, J=7.12 Hz), 4.21 (2H, q, J=7.15 Hz), 4.08 (2H, s), 3.50 (2H, s), 2.37 (1H, s) 1.45 (3H, t, J=7.12 Hz), 1.28 (3H, t, J=7.15 Hz). MS(ESI-TOF) calculated for C₁₇H₂₀N₄O₅ [M+H]⁺: calculated 333.15, found 334.09.

### Example 46. Synthesis of ethyl 2-((4-(thiophen-2-yl)-6-(carboxyethyl)-pyridine-2-yl)methylenenitrilo)-acetate 50

This compound **50** was synthesized from 4-(thiophen-2-yl)-6-bromomethyl-2-carboxyethylpyridine (WO2005/021538) using a method analogous to the synthesis described in the Example 3. The product was purified by column chromatography using silica gel as stationary phase and methanol:CH₂Cl₂ (5:95) as eluent. ¹H NMR (CDCl₃, δ ppm): 8.19 (1H, d, J=1.70 Hz), 7.79 (1H, d, J=1.70 Hz), 7.61 (1H, dd, J=1.05 and 3.70 Hz), 7.45 (1H, dd, J=1.05 and 5.05 Hz), 7.15 (1H, dd, J=3.70 and 5.05 Hz), 4.50 (2H, q, J=7.12 Hz), 4.20 (2H, q, J=7.15 Hz), 4.09 (2H, s), 3.51 (2H, s), 2.45 (1H, s), 1.45 (3H, t, J=7.12 Hz), 1.28 (3H, t, J=7.15 Hz). MS(ESI-TOF) calculated for C₁₇H₂₀N₂O₄S [M+H]⁺: calculated 349.11, found 349.99.

### Example 47. Synthesis of diethyl 6,6'-((((4-bromopyridine-2,6-diyl)bis(methylene))bis((2-ethoxy-2-oxoethyl)azanediyl))bis(methylene))bis(4-(furan-2-yl)picolinate) 51

A mixture of the compound **49** (0.16 g, 0.48 mmol), 2,6-dibromomethyl-4-bromopyridine (83 mg, 0.24 mmol; Acta Chem. Scand. 1988, Ser B, 42, 614), dry K₂CO₃ (0.13 g, 0.96 mmol) and dry MeCN (8 ml) was stirred for 4.5 hours at 55°C. The reaction mixture was filtered, the solid material washed with MeCN and the filtrate evaporated to dryness. The product was purified by column chromatography using silica gel as stationary phase and triethylamine:ethyl acetate:petroleum ether (1:69:30) as eluent. Yield: 0.19 g (95%) ¹H NMR (CDCl₃, δ ppm): 8.18 (2H, d, J=1.38 Hz), 8.05 (2H, J=1.38 Hz), 7.70 (2H, s), 7.58 (2 H, d, J=1.68 Hz), 6.98 (2 H, d, J=3.42 Hz), 6.54 (2H, dd, J=1.68 and 3.42 Hz), 4.48 (4H, q, J=7.11 Hz), 4.17 (4H, J=7.12 Hz), 4.10 (4H, s), 3.96 (4H, s), 3.50 (4H, s), 1.44 (6H, t, J=7.11 Hz), 1.27 (6H, t, J=7.12 Hz). MS(ESI-TOF) calculated for C₄₁H₄₄BrN₅O₁₀ [M+H]⁺: calculated 846.24 and 848.24, found 846.79 and 848.80.

### Example 48. Synthesis of diethyl 6,6'-((((4-bromopyridine-2,6-diyl)bis(methylene))bis((2-ethoxy-2-oxoethyl)azanediyl))bis(methylene))bis(4-(thiophen-2-yl)picolinate) 52

This compound **52** was synthesized from the compound **50** using a method analogous to the synthesis described in the Example 47. The product was purified by column chromatography using silica gel as stationary phase and ethanol:CH₂Cl₂ (gradient from 2:98 to 15:85) as eluent. Yield: 50%. ¹H NMR (CDCl₃, δ ppm): 8.15 (2H, s). 8.02 (2H, s), 7.71 (2H, s), 7.70 (2H, d, J=3.1 Hz), 7.42 (2H, J=4.8 Hz), 7.14 (2H, dd, J=3.1 and 4.8 Hz), 4.49 (4H, q, J=7.18 Hz), 4.18 (4H, q, J= 7.07 Hz), 3.96 (4H, s), 3.96 (4H, s), 3.50 (4H, s), 1.43 (6H, t, J=7.18 Hz), 1.27 (6H, t, J=7.07Hz). MS(ESI-TOF) calculated for C₄₁H₄₄BrN₅O₁₀S₂ [M+H]⁺: calculated 878.19 and 880.19, found 878.77 and 880,74. **Example 49.** Synthesis of diethyl 6,6'-((((4-((4-aminophenyl)ethynyl)pyridine-2,6-diyl)bis(methylene))bis((2-ethoxy-2-oxoethyl)azanediyl))bis(methylene))bis(4-(furan-2-yl)picolinate) **53**

A mixture of the compound **51** (0.18 g, 0.21 mmol) and 4-ethynylaniline (30 mg, 0.26 mmol) in dry triethylamine (1 ml) and tetrahydrofurane (2 ml) was de-aerated with argon. After addition of bis(triphenylphosphine)palladium(II) chloride (10 mg, 14 µmol) and Cul (6 mg, 28 µmol), the mixture was stirred for 20 hours at 55°C. After evaporation to dryness, The product was purified by column chromatography using silica gel as stationary phase and triethylamine:ethyl acetate:petroleum ether (first 1:69:30, then 1:79:20) as eluent. Yield: 0.14 g (74%). ¹H NMR (CDCl₃, δ ppm): 8.18 (2H, d, J=1.20 Hz), 8.12 (2H, d, J=1.20 Hz), 7.74 (2H, s), 7.52 (2H, d, J=1.5 Hz), 7.32 (2H, d, J=8.48 Hz), 6.99 (2H, d, J=3.32 Hz), 6.65 (2H, d, J=8.48Hz), 6.45 (2H, dd, J=1.5 and 3.32 Hz), 4.47 (4H, q, J=7.12 Hz), 4.17 (4H, q, J=7.12 Hz), 4.11 (4H. s), 3.98 (4H, s), 3.52 (4H, s), 1.43 (6H, t, J=7.12 Hz), 1.26 (6H, t, J=7.12 Hz). MS(ESI-TOF) calculated for C₄₉H₅₀N₆O₁₀ [M+H]⁺: calculated 883.34, found 883.90.

### Example 50. Synthesis of diethyl 6,6'-((((4-((4-aminophenyl)ethynyl)pyridine-2,6-diyl)bis(methylene))bis((2-ethoxy-2-oxoethyl)azanediyl))bis(methylene))bis(4-(thiophen-2-yl)picolinate) 54

This compound **54** was synthesized from the compound **52** using a method analogous to the synthesis described in the Example 49. Yield: 50%. ¹H NMR (CDCl₃, δ ppm): 8.14 (2H, s), 8.11 (2H, s), 7.61 (2H, d, J=3.40 Hz), 7.58 (2H, s), 7.30-7.33 (4 H, m), 7.03-7.07 (2H, m), 8.35 (2H, d, J=8.35 Hz), 4.47 (4H, q, J=7.09 Hz), 4.18 (4H, q, J=7.10 Hz), 4.13 (4H, s), 3.98 (4H, s), 3.52 (4H, s), 1.41 (6h, t, J=7.09 Hz), 1.26 (6H, t, J=7.10 Hz). MS(ESI-TOF) calculated for C₄₉H₅₀N₆O₈S₂ [M+H]⁺: calculated 915.32, found 915.05.

### Example 51. Synthesis of europium(III) chelate 55

A mixture of the compound **53** (0.13 g, 0.143 mmol) and 0.5M KOH in ethanol (9 ml) was stirred for 30 minutes at room temperature and water was added (1 ml). After stirring for 3 hours at room temperature, EtOH was evaporated. After addition of H₂O (3 ml), the mixture was stirred for 3 hours at room temperature, and the pH was adjusted to ca. 6.5 with 6M HCl.Europium(III) chloride (52 mg, 0.143 mmol) in H₂O (0.39 ml) was added within 10 minutes and the pH was maintained at 5-7 with solid NaHCO₃. After stirring for overnight at room temperature, the pH was raised to 8.5 with 1 M NaOH. The solution was triturated with tetrahydrofurane, the precipitate centrifuged and washed with tetrahydrofurane. The product was used for the next step without further purification. Conditions for HPLC run: see example 16. R_{f}(HPLC)=24.9 min. UV/VIS=323 and 355 (sh) nm. MS(ESI-TOF) calculated for C₄₁H₃₀EuN₆NaO₁₀ [M+2H]⁺ cal 944.13 found 945.73

### Example 52. Synthesis of europium(III) chelate 56

This compound **56** was synthesized from the compound **54** using a method analogous to the synthesis described in the Example 51. Conditions for HPLC run: see example 16. R_{f}(HPLC)=26.3 min. UV/VIS=325 and 356 (sh) nm. MS(ESI-TOF) calculated for C₄₁H₃₀EuN₆NaO₈S₂ [M+2H]⁺: calculated 976.09, found 976.88.

### Example 53. Synthesis of europium(III) labeling chelate 57

This compound **57** was synthesized from the chelate **55** using a method analogous to the synthesis described in the Example 15. Conditions for HPLC run: see example 16. R_{f}(HPLC)=33.4. UV/VIS=325 nm. MS(ESI-TOF) calculated for C₄₂H₂₈EuN₆NaO₁₀S [M+H]⁺ calculated 985.08, found 984.77.

### Example 54. Synthesis of europium(III) labeling chelate 58

This compound **58** was synthesized from the chelate **56** using a method analogous to the synthesis described in the Example 15. Conditions for HPLC run: see example 16. R_{f}(HPLC)=34.7. UV/VIS=325 nm. MS(ESI-TOF) calculated for C₄₂H₂₈EuN₆NaO₈S₃ [M+H]⁺ calculated 1017.04, found 1017.21.

### Example 55. Synthesis of europium(III) chelate 59

This compound **59** was synthesized from the chelate **57** using a method analogous to the synthesis described in the Example 16. Conditions for HPLC run: see example 16. R_{f}(HPLC)=23.2. UV/VIS=321 nm.

### Example 56. Synthesis of europium(III) chelate 60

This compound **60** was synthesized from the chelate **58** using a method analogous to the synthesis described in the Example 16. Conditions for HPLC run: see example 16. R_{f}(HPLC)=24.9. UV/VIS=322 nm.

### Example 57. Synthesis of diethyl 2,2'-((4-bromo-1,3-phenylene)bis(oxy))diacetate (61)

4-bromoresorcinol (1.00 g, 5.29 mmol) was dissolved in DMF (20 ml, dry). Anhydrous K₂CO₃ (4.39 g, 31.74 mmol) and ethyl bromoacetate (2.30 ml, 15.87 mmol) were added. Mixture was stirred overnight at 40°C under argon atmosphere. Water (30 ml) was added and the mixture was extracted with ethyl acetate (1x 20 ml, 2x10 ml). Combined organic extracts were washed with water (2x10 ml), dried over Na₂SO₄ and concentrated. Crude product was purified by column chromatography using silica gel as stationary phase and dichloromethane as eluent. Product was a white solid. Yield: 1.70 g (89%) 1H NMR (CDCl3, δ ppm): 7.43 (1H, d, J=8.7Hz), 6.48 (1H, d, J=2.7Hz), 6.40 (1H, dd, J=8.7Hz, J=2.7Hz), 4.66 (2H, s), 4.57 (2H, s), 4.27 (4H, m) 1.30 (6H, m).

### Example 58. Synthesis of diethyl 2,2'-((4-((trimethylsilyl)ethynyl)-1,3-phenylene)bis(oxy))diacetate (62)

Compound **61** (1.56 g, 4.33 mmol) was dissolved in DMF (3 ml, dry) and the solution was placed in a microwave reaction vial. Diethyl amine (9 ml, dry), Pd(PPh₃)₂Cl₂ (152.0 mg, 0.217 mmol), Cul (41.2 mg, 0.217 mmol) and PPh₃ (113.6 mg, 0.433 mmol) were added and the vial was sealed in an argon atmosphere. Trimethylsilyl acetylene (925 µl, 6.50 mmol) was added through a septum and the mixture was stirred at 100°C for 30 minutes using microwave heating. Reaction mixture was filtrated through silica gel using dichloromethane as eluent and the filtrate was evaporated to dryness. The crude product was dissolved in dichloromethane and purified by column chromatography using silica gel as stationary phase and ethyl acetate:petroleum ether (10:90) as eluent. Yield: 0.99 g (61%). 1H NMR (CDCl3, δ ppm): 7.36 (1H, dd, J=1.35Hz, J=7.55Hz), 6.43 (2H, m), 4.67 (2H, s), 4.58 (2H, s), 4.27 (4H, m), 1.30 (6H, m), 0.25 (9H, s).

### Example 59. Synthesis of diethyl 2,2'-((4-ethynyl-1,3-phenylene)bis(oxy))diacetate (63)

Compound **62** (398.9 mg, 1.054 mmol) was dissolved in dichloromethane (10 ml, dry). Tetrabutyl ammoniumfluoride (330.7 mg, 1.265 mmol) was added and the mixture was stirred in argon atmosphere at room temperature for 1 h 30 min. Mixture was washed with 10% citric acid solution (5 ml), and water (4x10 ml). The organic phase was dried over Na₂SO₄ and evaporated to dryness. Crude product was purified by column chromatography using silica gel as stationary phase and ethyl acetate:petroleum ether (15:85) as eluent. Yield 259.2 mg (80%). 1H NMR (CDCl3, δ ppm): 7.40 (1H, d, J=8.35Hz), 6.45 (1H, d, J=2.4Hz), 6.43 (1H, dd, J=5.25Hz, J=2.4Hz), 4.70 (2H, s), 4.59 (2H, s), 4.27 (4H, m) 3.24 (1H, s), 1.29 (6H, m).

### Example 60. Synthesis of N-(4-bromo-3-methoxyphenyl)-2,2,2-trifluoroacetamide (64)

4-bromo-3-methoxyaniline (2.00 g, 9.90 mmol) was dissolved in THF (15 ml). The solution was added to ice cold trifluoroacetic anhydride (15 ml, 107.8 mmol) and the mixture was stirred 20 minutes on an ice-bath. Mixture was poured to ice water and stirring continued for 10 minutes. The mixture was extracted with dichloromethane (2x20 ml). Combined organic layers were washed with 5% NaHCO₃ (5x40 ml) and water (30 ml). Washed organic layers were dried on Na₂SO₄, filtered, and evaporated to dryness. The product (3.08, >99%) was used without further purifications.

### Example 61. Synthesis of N-(4-bromo-3-hydroxyphenyl)-2,2,2-trifluoroacetamide (65)

Compound **65** was synthesized from compound **64** (3.08 g, 10mmol) using method analogous to the synthesis in example 9. Yield 0.51 g (17%). ¹H NMR (DMSO, δ ppm): 11.24 (1H, s), 10.51 (1H, s), 7.48 (2H, m), 7.05 (1H, dd, J=2.5 and 8.7 Hz).

### Example 62. Synthesis of ethyl 2-(2-bromo-5-(2,2,2-trifluoroacetamido)phenoxy)acetate (66)

Compound **66** was synthesized from compound **65** (0.51 g, 1.78mmol) using method analogous to the synthesis in example 10. CH₃CN was used as solvent. Reaction time at 40°C was 18h. Yield 0.47 g (71%). ¹H NMR (CDCl₃, δ ppm): 7.96 (1H, s), 7.52 (1H, d, J=8.5 Hz), 7.38 (1H, d, J=2.3 Hz), 6.91 (1H, dd, J=2.35 and 8.55 Hz), 4.72 (2H, s), 4.28 (2H, q, J=7.15 Hz), 1.32 (3H, t, J=7.15 Hz).

### Example 63. Synthesis of ethyl 2-(5-(2,2,2-trifluoroacetamido)-2-((trimethylsilyl)ethynyl)phenoxy) acetate (67)

Compound **67** was synthesized from compound **66** (0.46 g, 1.24 mmol) using method analogous to the synthesis in example 11. Reaction time in the microwave at 100°C was 30 minutes. Yield 0.37 g (77%). ¹H NMR (CDCl₃, δ ppm): 7.92 (1H, s), 7.43 (1H, d, J=8.3 Hz), 7.34 (1H, d, J=1.9 Hz), 6.98 (1H, dd, J=1.9 and 8.3 Hz), 4.72 (2H, s), 4.29 (2H, q, J=7.15 Hz), 1.31 (3H, t, J=7.15 Hz), 0.26 (9H, s).

### Example 64. Synthesis of ethyl 2-(2-ethynyl-5-(2,2,2-trifluoroacetamido)phenoxy)acetate (68)

Compound **68** was synthesized from compound **67** (0.37 g, 0.96 mmol) using method analogous to the synthesis in example 12. Yield 0.30 g (99%).

### Example 65. Synthesis of ethyl 2-(2-((2,6-bis(hydroxymethyl)pyridin-4-yl)ethynyl)-5-(2,2,2-trifluoroacetamido)phenoxy)acetate (69)

Compound **69** was synthesized from compound **68** (0.37 g, 0.96 mmol) using method analogous to the synthesis in example 30. Yield 0.19 g (44%).

### Example 66. Synthesis of ethyl 2-(2-((2,6-bis(bromomethyl)pyridin-4-yl)ethynyl)-5-(2,2,2-trifluoroacetamido)phenoxy)acetate (70)

Compound **70** was synthesized from compound **69** (0.19 g, 0.42 mmol) using method analogous to the synthesis in example 31. Yield 0.22 g (90%). ¹H NMR (CDCl₃, δ ppm): 7.94 (1H, s), 7.51 (1H, d, J=8.3 Hz), 7.49 (2H, s), 7.44 (1H, d, J=1.85 Hz), 7.02 (1H, dd, J=1.9 and 8.3 Hz), 4.78 (2H, s), 4.53 (4H, s), 4.31 (2H, q, J=7.15 Hz), 1.33 (3H, t, J=7.15 Hz).

### Example 67. Synthesis of diethyl 6,6'-((((4-((2-(2-ethoxy-2-oxoethoxy)-4-(2,2,2-trifluoroacetamido)phenyl)ethynyl)pyridine-2,6-diyl)bis(methylene))bis((2-ethoxy-2-oxoethyl)azanediyl))bis(methylene))bis(4-bromopicolinate) (71)

Compound **71** was synthesized from compound **70** (0.19 g, 0.42 mmol) and compound **5** (0.25 g, 0.73 mmol) using method analogous to the synthesis in example 4. Reaction time in 55°C was 20 hours. Yield 0.23 g (57%). MS(MALDI) calculated for C₄₇H₄₉Br₂F₃N₆O₁₂ [M+H]⁺: calculated 1107.18 found 1107.62.

### Example 68. Synthesis of compound 72

Compound **72** was synthesized from compound **71** (0.11 g, 0.10 mmol) and compound **63** (75 mg, 0.25 mmol) using method analogous to the synthesis in example 33. Yield 140 mg (87%)

### Example 69. Synthesis of the europium(III) chelate 73

Compound **73** was synthesized from compound **72** (0.14 g, 0.09 mmol) using method analogous to the synthesis in example 36. The product was used for the next step without further purification. Conditions for HPLC run: see example 16. R_{f}(HPLC)=14.5 min. UV/VIS=350 nm.

### Example 70. Synthesis of the europium(III) labeling chelate 74

Compound **74** was synthesized from compound **73** (0.1 g, 0.07 mmol) using method analogous to the synthesis described in the Example 15. Conditions for HPLC run: see example 16. R_{f}(HPLC)=18.7 min. UV/VIS= 340 nm.

### Example 71. Synthesis of the europium(III) chelate 75

Compound **75** was synthesized from compound **74** using method analogous to the synthesis described in the Example 16. Conditions for HPLC run: see example 16. R_{f}(HPLC)=14.5 min. UV/VIS=351 nm.

### Example 72. Photo-physical properties of novel chelates conjugated to taurine (chelates 19, 46, 47, 48, 59 and 75)

The prepared isothiocyante activated chelates (**19**, **43**, **44**, **45**, **57** and **75**) were conjugated to taurine as described above in Example 16. The products were purified with semi-preparative reversed phase HPLC (RP-18 column). After the product fractions were evaporated the residues were dissolved in 50 mM TRIS buffer.

The measured photo-physical properties excitation wavelengths (λ_{exc}), luminescence decay times (τ), molar absorptivities (ε), estimated luminescence yields (εΦ) of the novel chelates (**19**, **46**, **48**, and **59**) in 50 mM TRIS buffer (pH 7.75) are in the Table 3.

**Table 3.**

| **Compound** | **ε/M¹cm⁻¹** | **Estimated εΦ** | **τ/ms** | **λ_{exc}/nm** |
|---|---|---|---|---|
| **19** | 83000 | 14100 | 0,98 | 323 |
| **46** | 64000 | 16400 | 0,70 | 343 |
| **48** | 69000 | 13800 | 0,77 | 340 |
| **59** | 139000 | 12600 | 0,94 | 32D |
| **75** | 82000 | 16400 | 0,79 | 347 |

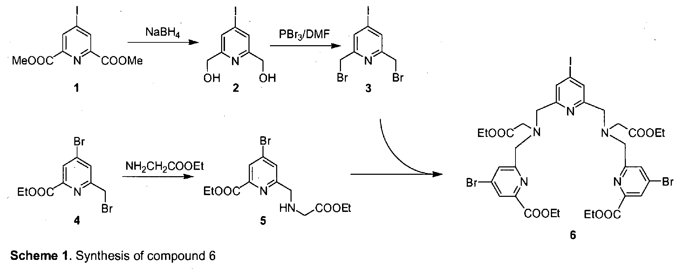

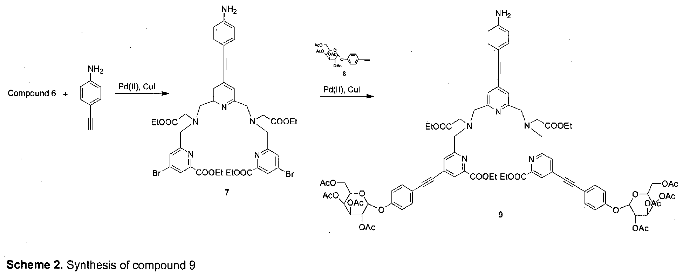

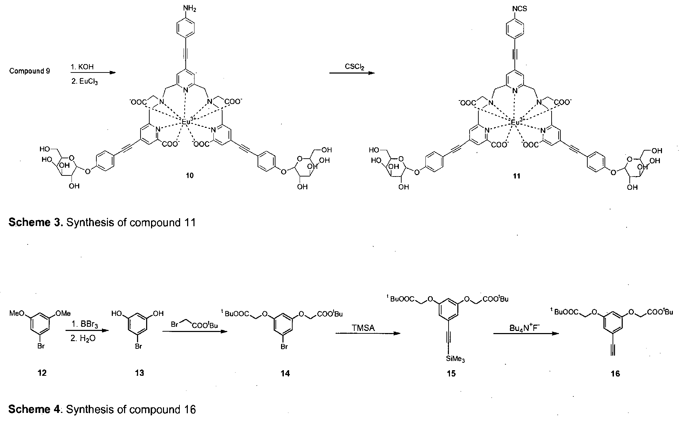

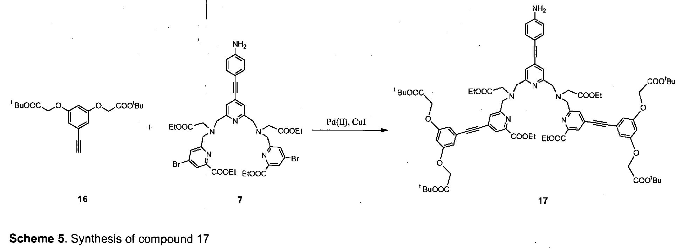

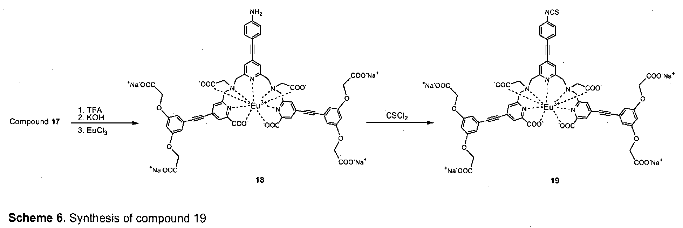

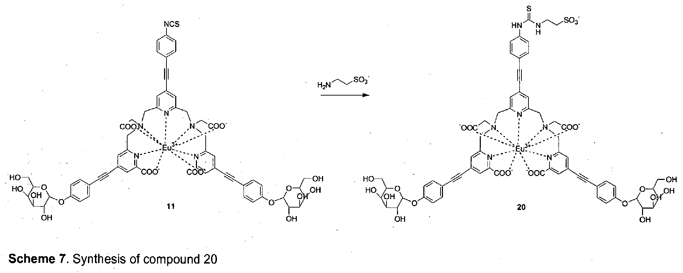

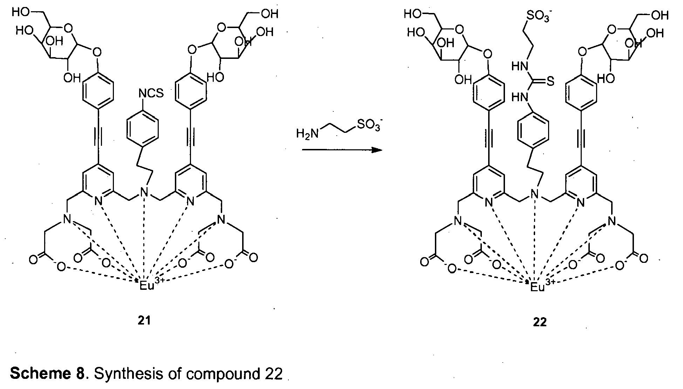

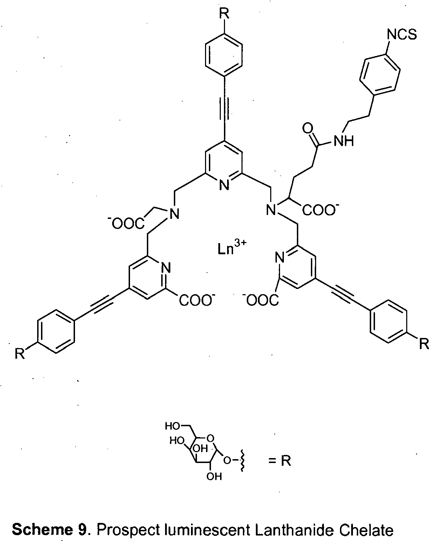

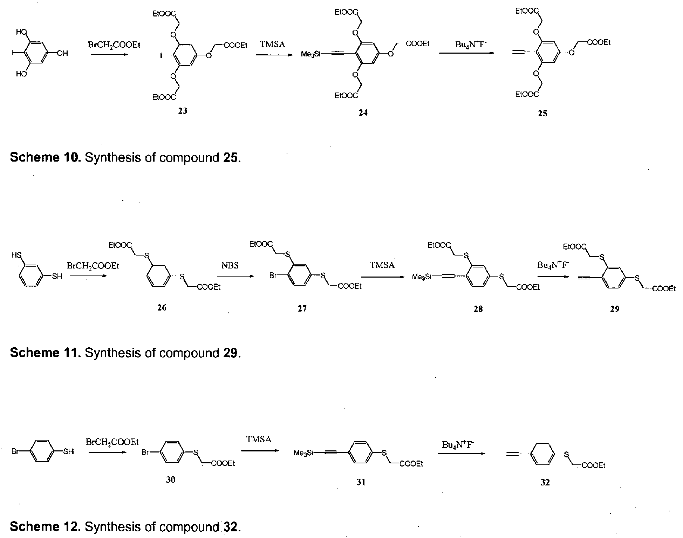

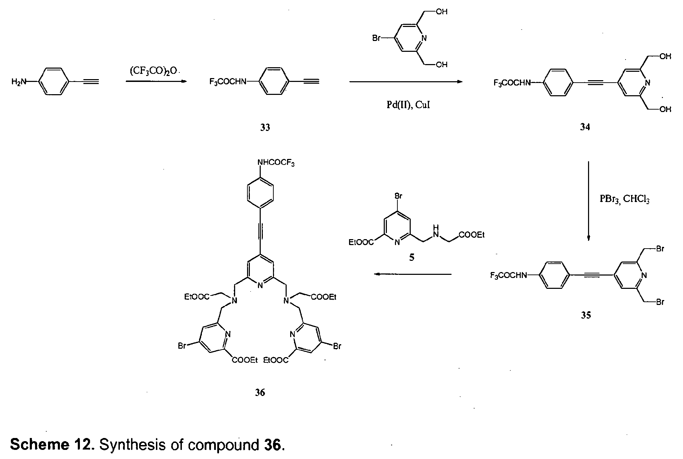

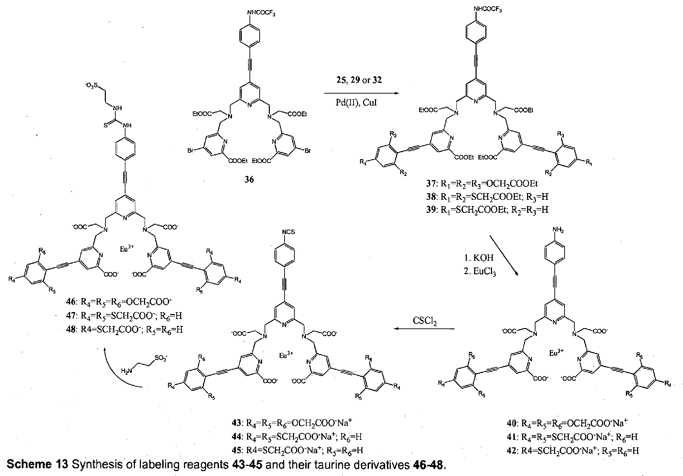

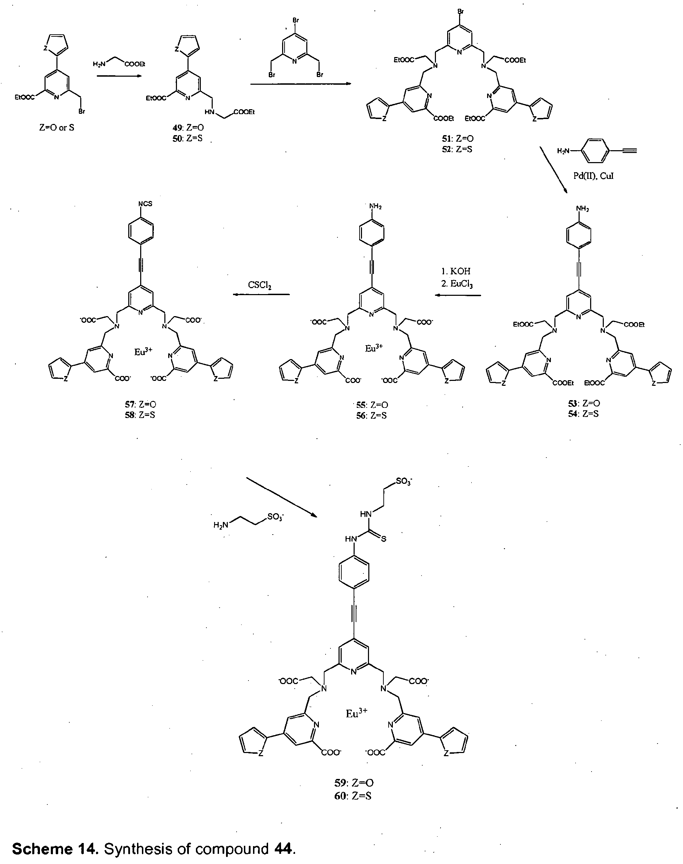

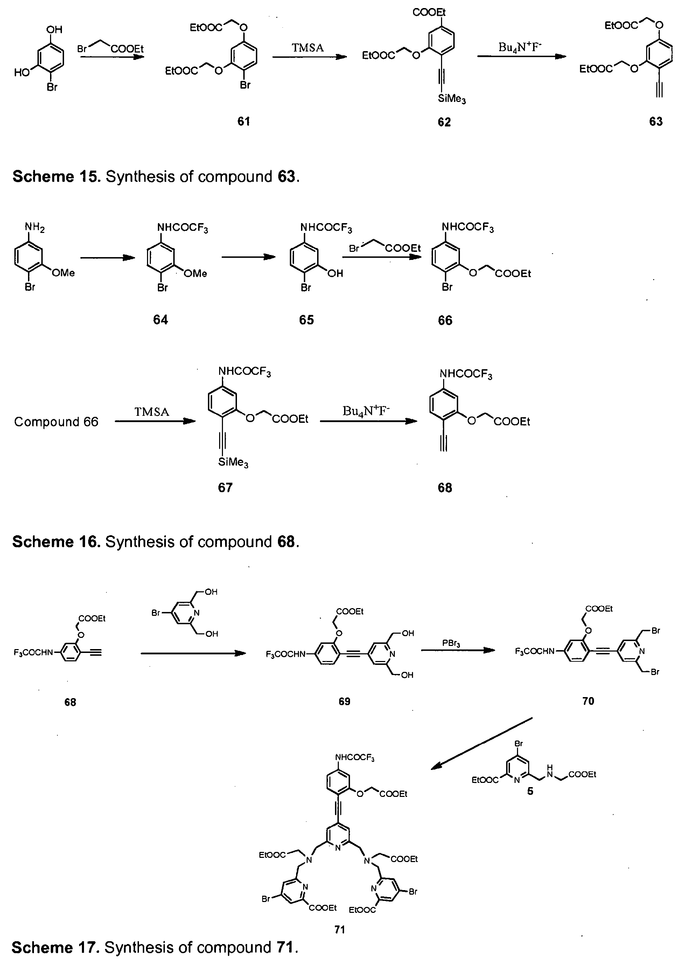

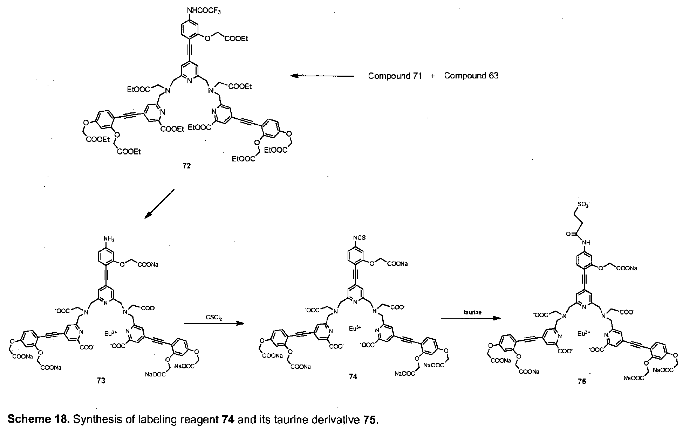

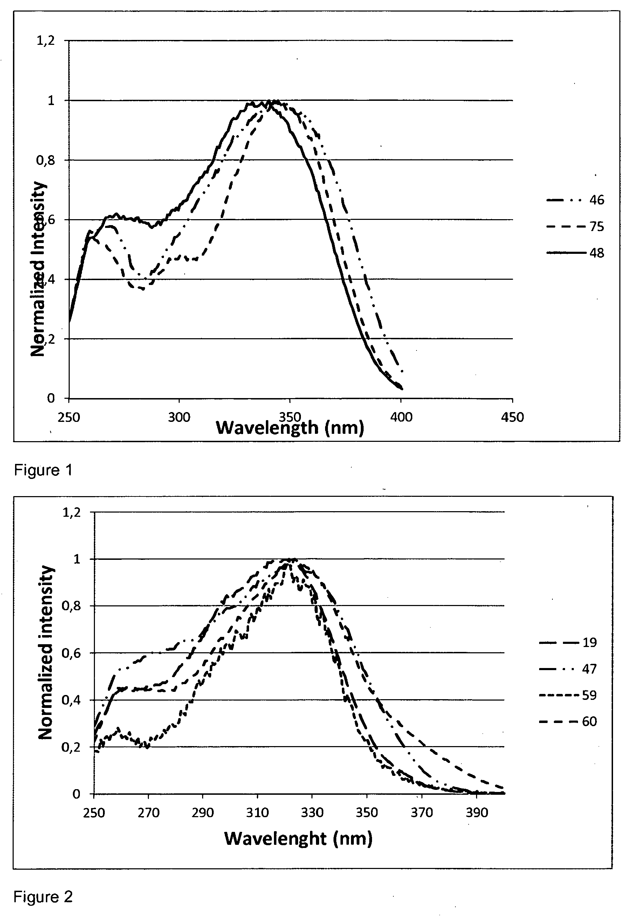

Excitation maxima of chelates **46, 48** and **75** are presented in Figure 1 as well as chelates **19, 47, 59** and 60 in the Figure 2, to show the broad excitation area offering the possibility to use cheap LED based excitation possibilities.

### Example 73. Labelling of antibody with chelates 19, 43, 45 and 74

The Tnl labeled antibodies were prepared as described in Example 17.

The measured photo-physical properties excitation wavelengths (λ_{exc}), luminescence decay times (τ), molar absorptivities (ε), luminescence yields (εΦ) of the labelled cTnls with the chelates **19**, **43**, **45** and **74** in 50 mM TRIS buffer (pH 7.75) in the Table 4.

Dry measurements (**19** (dry), **43** (dry), **45** (dry) and **74** (dry)) represents estimated luminescence yields based on the signal measurements after dry immunoassay done as described in the Example 18.

**Table 4.**

| **Compound** | **ε/M⁻¹cm⁻¹** | **Estimated εφ** | **τ/ms** | **λ_{exc}/nm** |
|---|---|---|---|---|
| **19** | 70000 | 9100 | 0,90 | 323 |
| **19 (dry)** | | 10200 | | |
| **43** | 78000 | 11400 | 0,69 | 344 |
| **43 (dry)** | | 20700 | | |
| **45** | 88000 | 6800 | 0,66 | 343 |
| **45 (dry)** | | 9000 | | |
| **74** | 110000 | 11600 | 0,71 | 350 |
| **74 (dry)** | | 13700 | | |

## Claims

1. A luminescent lanthanide chelate of formula (I) 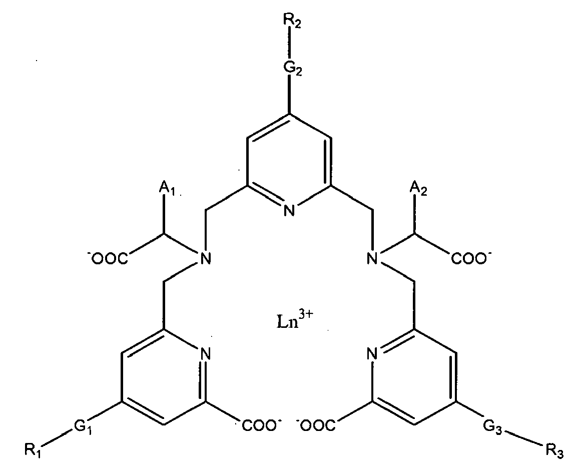 wherein
each of G₁, G₂ and G₃ is independently selected from i) a conjugating group and ii) a single bond, provided that at least one of G₁, G₂ and G₃ is independently a conjugating group;
each of R₁, R₂, and R₃ is independently selected from i) a reactive group Z, ii) a hydrophilic group, and iii) hydrogen, or the respective group, R₁, R₂, and R₃, is absent;
each of A₁ and A₂ is independently selected from i) a reactive group Z, ii) a hydrophilic group, and iii) hydrogen or C₁₋₆-alkyl;
and wherein at least one of R₁, R₂, R₃, A₁ and A₂ is a reactive group Z, and
Ln³⁺ is a lanthanide ion.

2. The luminescent lanthanide chelate according claim 1, wherein the conjugating group is consisting of one, two or three moieties arranged so as to be conjugated with each other, each moiety being selected from ethenylene (-CH=CH-), ethynediyl (-C≡C-), carbonyl (-C(=O)-), biradicals of (hetero)aromatic ring or ring systems (-Het/Ar-), e.g. phenylene, biphenylene, naphthylene, pyridylene, pyrazinylene, pyrimidinylene, pyridazinylene, furylene, thienylene, pyrrolylene, imidazolylene, pyrazolylene, thiazolylene, isothiazolylene, oxazolylene, isoxazolylene, fyrazanylene, 1,2,4-triazol-3,5-ylene, and oxadiazolylene, which each may be unsubstituted, mono-R₄-substituted, di-R₄,R₅-substituted, tri-R₄,R₅,R₆-substituted, tetra-R₄,R₅,R₆,R₇-substituted, or penta-R₄,R₅,R₆,R₇,R₈-substituted, wherein each of such possible substituents R₄, R₅, R₆, R₇, and R₈ independently are selected from C₁₋₁₂-alkyl, -(CH₂)₀₋₆COOH, -(CH₂)₀₋₆COO⁻, -(CH₂)₀₋₆SO₃H, -(CH₂)₀₋₆SO₃⁻,-NHC(=O)R₁₀, -NCH₃C(=O)R₁₀, -C(=O)NHR₁₀, -C(=O)NCH₃R₁₀,-NHC(=O)NHR₁₀, -NHC(=S)NHR₁₀, -C(=O)R₁₀, -F, -Cl, -Br, -I, hydroxyl (-OH), mercapto (-SH), -OR₉,-SR₉, and a hydrophilic group, and wherein R₉ is selected from -CF₃, -C₁₋₁₂-alkyl, -(CH₂)₁₋₆COOH, -(CH₂)₁₋₆COO⁻,-(CH₂)₁₋₆SO₃H, -(CH₂)₁₋₆SO₃⁻, -NHC(=O)R₁₀, -NCH₃C(=O)R₁₀,-C(=O)NHR₁₀, -C(=O)NCH₃R₁₀, -NHC(=O)NHR₁₀, -NHC(=S)NHR₁₀, -C(=O)R₁₀ and a hydrophilic group, wherein R₁₀ is selected from C₁₋₁₂-alkyl,-(CH₂)₁₋₆COOH, -(CH₂)₁₋₆COO⁻, -(CH₂)₁₋₆SO₃H, and -(CH₂)₁₋₆SO₃⁻ and a hydrophilic group.

3. The luminescent lanthanide chelate according to any one of the preceding claims, wherein at least two of G₁, G₂ and G₃ are independently a conjugating group.

4. The luminescent lanthanide chelate according to any one of the preceding claims, wherein each of G₁, G₂ and G₃ independently is a conjugating group.

5. The luminescent lanthanide chelate according to any one of the preceding claims, wherein the reactive group Z comprises azido (-N₃), alkynyl (-C=CH), alkylene (-CH=CH₂), amino (-NH₂), aminooxy (-O-NH₂), carboxyl (-COOH), aldehyde (-CHO), mercapto (-SH), maleimido groups or activated derivatives thereof, including isocyanato (-NCO), isothiocyanato (-NCS), diazonium (-N⁺N), bromoacetamido, iodoacetamido, reactive esters, pyridyl-2-dithio, or 6-substituted 4-chloro-1,3,5-triazin-2-ylamino.

6. The luminescent lanthanide chelate according to any one of the preceding claims, wherein the reactive group Z comprises a spacer which is a distance-making biradical, the said spacer is formed of one to five moieties, each moiety is selected from the group consisting of phenylene, alkylene containing 1-10 carbon atoms, an ethynediyl (-C≡C-), an ether (-O-), a thioether (-S-), a disulfide (-S-S-), an amide (-C(=O)-NH-, -NH-C(=O)-, -C(=O)-NCH₃- and -NCH₃-C(=O)-), a thiourea (-NH-C(=S)-NH-) and a triazole.

7. The luminescent lanthanide chelate according to any one of the preceding claims, wherein the conjugating groups independently are selected from phenylethynyl, phenyl, thienyl and furyl, which each may be substituted.

8. The luminescent lanthanide chelate according to any one of the preceding claims, wherein the hydrophilic group comprises monosaccharides or disaccharides.

9. The luminescent lanthanide chelate according to any one of the preceding claims, wherein the hydrophilic group comprises a spacer which is a distance-making biradical, the said spacer is formed of one to five moieties, each moiety is selected from the group consisting of phenylene, alkylene containing 1-10 carbon atoms, an ethynediyl (-C≡C-), an ether (-O-), a thioether (-S-), a disulfide (-S-S-), an amide (-C(=O)-NH-, -NH-C(=O)-, -C(=O)-NCH₃- and -NCH₃-C(=O)-), a thiourea (-NH-C(=S)-NH-) and a triazole.

10. A luminescent lanthanide chelate according to any one of the preceding claims
(i) wherein each of G₁ and G₃ is 3,5-bis(carboxymethoxy)phenylethynyl and G₂ is phenylethynyl, each of -A₁ and -A₂ is -H, each of R₁ and R₃ is -H, and R₂ is a reactive group; or
(ii) wherein each of G₁, G₂ and G₃ is phenylethynyl, each of -A₁ and -A₂ is -H, each of R₁ and R₃ is a hydrophilic group, and R₂ is a reactive group; or
(iii) wherein each of G₁, G₂ and G₃ is phenylethynyl, one of -A₁ and -A₂ is -H, and the other of -A₁ and -A₂ is a reactive group including a spacer, and each of R₁, R₂ and R₃ is a hydrophilic group; or
(iv) wherein each of G₁ and G₃ are 4-(carboxymethylthio)phenylethynyl and G₂ is phenylethynyl, each of -A₁ and -A₂ is -H, each of R₁ and R₃ is -H, and R₂ is a reactive group; or
(v) wherein each of G₁ and G₃ are 2,4-bis(carboxymethylthio)phenylethynyl and G₂ is phenylethynyl, each of -A₁ and -A₂ is -H, each of R₁ and R₃ is -H, and R₂ is a reactive group; or
(vi) wherein each of G₁ and G₃ are 2,4,6-tris(carboxymethoxy)phenylethynyl and G₂ is phenylethynyl, each of -A₁ and -A₂ is -H, each of R₁ and R₃ is -H, and R₂ is a reactive group; or
(vii) wherein each of G₁ and G₃ are furan-2-yl and G₂ is phenylethynyl, each of - A₁ and -A₂ is -H, each of R₁ and R₃ is -H, and R₂ is a reactive group; or
(viii) wherein each of G₁ and G₃ are thien-2-yl and G₂ is phenylethynyl, each of - A₁ and -A₂ is -H, each of R₁ and R₃ is -H, and R₂ is a reactive group; or
(ix) wherein each of R₁-G₁ and R₃-G₃ are (2,4-di(NaOOC-CH₂-O-)phenyl)ethynyl, G₂ is (2-(NaOOC-CH₂-O-)phenyl)ethynyl, each of -A₁ and -A₂ is -H, and R₂ is a reactive group.

11. The luminescent lanthanide chelate according to any one of the preceding claims, wherein the lanthanide ion, Ln³⁺, is selected from europium(III), terbium(III), dysprosium(III) and samarium(III), preferably europium(III).

12. A detectable molecule comprising a biospecific binding reactant conjugated to a luminescent lanthanide chelate of the formula (I) as defined in any one of claims 1-11.

13. The detectable molecule according to claim 12, wherein the biospecific binding reactant is selected from an antibody, an antigen, a receptor ligand, a specific binding protein, a DNA probe, a RNA probe, an oligopeptide, an oligonucleotide, a modified oligonucleotide, a modified polynucleotide, a protein, an oligosaccaride, a polysaccharide, a phospholipid, a PNA, a steroid, a hapten, a drug, a receptor binding ligand, and lectine.

14. The detectable molecule according to any of claims 12 or 13, wherein the biospecific binding reactant is an antibody.

15. A lanthanide chelating ligand of the formula (II), 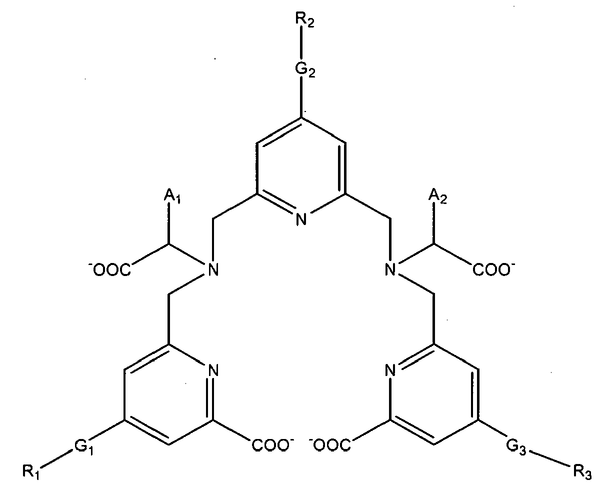 wherein each of G₁, G₂, G₃, R₁, R₂, R₃, A₁ and A₂ represents the groups G₁, G₂, G₃, R₁, R₂, R₃, A₁ and A₂ as defined in any one of claims 1-11.

16. A method of carrying out a biospecific binding assay, said method comprising the steps of:
a) forming a biocomplex between an analyte and a biospecific binding reactant labelled by lanthanide chelate according to any one of claims 1-11;
b) exciting said biocomplex with radiation having an excitation wavelength, thereby forming an excited biocomlex; and
c) detecting emission radiation emitted from said excited biocomplex.

17. Use of a detectable molecule according to any one of claims 12-14 in a specific bioaffinity based binding assay utilizing time-resolved fluorometric determination of a specific luminescence.

18. A solid support material conjugated with a luminescent lanthanide chelate of the formula (I) according to any of the claims 1-11 or a lanthanide chelating ligand of formula (II) according to claim 15.

## Patentansprüche

1. Lumineszierendes Lanthanidchelat der Formel (I): 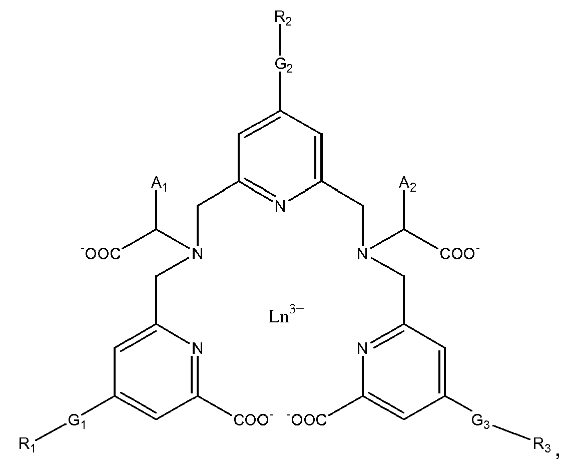 wobei
jedes von G₁, G₂ und G₃ unabhängig aus i) einer konjugierenden Gruppe und ii) einer Einfachbindung ausgewählt ist, vorausgesetzt, dass mindestens eines von G₁, G₂ und G₃ unabhängig eine konjugierende Gruppe ist;
jedes von R₁, R₂ und R₃ unabhängig aus i) einer reaktiven Gruppe Z, ii) einer hydrophilen Gruppe und iii) Wasserstoff ausgewählt ist oder die jeweilige Gruppe R₁, R₂ und R₃ fehlt;
jedes von A₁ und A₂ unabhängig aus i) einer reaktiven Gruppe Z, ii) einer hydrophilen Gruppe und iii) Wasserstoff oder C₁₋₆-Alkyl ausgewählt ist;
und wobei mindestens eines von R₁, R₂, R₃, A₁ und A₂ eine reaktive Gruppe Z ist, und
Ln³⁺ ein Lanthanidion ist.

2. Lumineszierendes Lanthanidchelat nach Anspruch 1, wobei die konjugierende Gruppe aus einer, zwei oder drei Einheiten besteht, die derart angeordnet sind, dass sie miteinander konjugiert werden, wobei jede Einheit aus Ethenylen (-CH=CH-), Ethindiyl (-C≡C-), Carbonyl (-C(=O)-), Biradikale von (hetero)aromatischen Ring- oder Ringsystemen (-Het/Ar-), z. B. Phenylen, Biphenylen, Naphthylen, Pyridylen, Pyrazinylen, Pyrimidinylen, Pyridazinylen, Furylen, Thienylen, Pyrrolylen, Imidazolylen, Pyrazolylen, Thiazolylen, Isothiazolylen, Oxazolylen, Isoxazolylen, Fyrazanylen, 1,2,4-Triazol-3,5-ylen und Oxadiazolylen ausgewählt ist, die jeweils unsubstituiert, mono-R₄-substitutiert, di-R₄,R₅-substitutiert, tri-R₄,R₅,R₆-substitutiert, tetra-R₄,R₅,R₆,R₇-substitutiert oder penta-R₄,R₅,R₆,R₇,R₈-substitutiert sein können, wobei jeder der derartigen möglichen Substituenten R₄, R₅, R₆, R₇ und R₈ unabhängig aus C₁₋₁₂-Alkyl, -(CH₂)₀-₆COOH, -(CH₂)₀-₆COO-, -(CH₂)₀-₆SO₃H, -(CH₂)₀-₆SO₃-,-NHC(=O)R₁₀, -NCH₃C(=O)R₁₀, -C(=O)NHR₁₀, -C(=O)NCH₃R₁₀,-NHC(=O)NHR₁₀, -NHC(=S)NHR₁₀, -C(=O)R₁₀, -F, -Cl, -Br, -I, Hydroxyl (-OH), Mercapto (-SH), -OR₉,-SR₉ und einer hydrophilen Gruppe ausgewählt ist und wobei R₉ aus -CF₃, -C₁-₁₂-Alkyl, -(CH₂)₁-₆COOH, -(CH₂)₁-₆COO⁻,-(CH₂)₁-₆S_{O3}H, -(CH₂)₁-₆SO₃⁻, -NHC(=O)R₁₀, -NCH₃C(=O)R₁₀,-C(=O)NHR₁₀, -C(=O)NCH₃R₁₀, -NHC(=O)NHR₁₀, -NHC(=S)NHR₁₀, -C(=O)R₁₀ und einer hydrophilen Gruppe ausgewählt ist, wobei R₁₀ aus C₁₋₁₂-Alkyl,-(CH₂)₁-₆COOH, -(CH₂)₁-₆COO⁻, -(CH₂)₁-₆SO₃H und -(CH₂)₁-₆SO₃⁻ und einer hydrophilen Gruppe ausgewählt ist.

3. Lumineszierendes Lanthanidchelat nach einem der vorhergehenden Ansprüche, wobei mindestens zwei von G₁, G₂ und G₃ unabhängig eine konjugierende Gruppe sind.

4. Lumineszierendes Lanthanidchelat nach einem der vorhergehenden Ansprüche, wobei jedes von G₁, G₂ und G₃ unabhängig eine konjugierende Gruppe ist.

5. Lumineszierendes Lanthanidchelat nach einem der vorhergehenden Ansprüche, wobei die reaktive Gruppe Z Azido- (-N₃), Alkinyl- (-C≡CH), Alkylen-(-CH=CH₂), Amino- (-NH₂), Aminooxy- (-O-NH₂), Carboxyl- (-COOH), Aldehyd-(-CHO), Mercapto- (-SH), Maleinimidogruppen oder aktivierte Derivate davon umfasst, einschließlich Isocyanato (-NCO), Isothiocyanato (-NCS), Diazonium (-N⁺N), Bromacetamido, lodacetamido, reaktive Ester, Pyridyl-2-dithio oder 6-substitutiertes 4-Chlor-1,3,5-triazin-2-ylamino.

6. Lumineszierendes Lanthanidchelat nach einem der vorhergehenden Ansprüche, wobei die reaktive Gruppe Z einen Spacer umfasst, bei dem es sich um ein Abstand schaffendes Biradikal handelt, wobei der Spacer von ein bis fünf Einheiten gebildet wird, wobei jede Einheit aus der Gruppe bestehend aus Phenylen, Alkylen, das 1-10 Kohlenstoffatome enthält, einem Ethindiyl (-C≡C-), einem Ether (-O-), einem Thioether (-S-), einem Disulfid (-S-S-), einem Amid (-C(=O)-NH-, -NH-C(=O)-, -C(=O)-NCH₃- und -NCH₃-C(=O)-), einem Thioharnstoff (-NH-C(=S)-NH-) und einem Triazol ausgewählt ist.

7. Lumineszierendes Lanthanidchelat nach einem der vorhergehenden Ansprüche, wobei die konjugierenden Gruppen unabhängig aus Phenylethinyl, Phenyl, Thienyl und Furyl ausgewählt sind, die jeweils substituiert sein können.

8. Lumineszierendes Lanthanidchelat nach einem der vorhergehenden Ansprüche, wobei die hydrophile Gruppe Monosaccharide oder Disaccharide umfasst.

9. Lumineszierendes Lanthanidchelat nach einem der vorhergehenden Ansprüche, wobei die hydrophile Gruppe einen Spacer umfasst, bei dem es sich um ein Abstand schaffendes Biradikal handelt, wobei der Spacer von ein bis fünf Einheiten gebildet wird, wobei jede Einheit aus der Gruppe bestehend aus Phenylen, Alkylen, das 1-10 Kohlenstoffatome enthält, einem Ethindiyl (-C≡C-), einem Ether (-O-), einem Thioether (-S-), einem Disulfid (-S-S-), einem Amid (-C(=O)-NH-, -NH-C(=O)-, -C(=O)-NCH₃- und -NCH₃-C(=O)-), einem Thioharnstoff (-NH-C(=S)-NH-) und einem Triazol ausgewählt ist.

10. Lumineszierendes Lanthanidchelat nach einem der vorhergehenden Ansprüche,
(i) wobei jedes von G₁ und G₃ 3,5-Bis(carboxymethoxy)phenylethinyl ist und G₂ Phenylethinyl ist, jedes von -A₁ und -A₂ -H ist, jedes von R₁ und R₃ -H ist und R₂ eine reaktive Gruppe ist; oder
(ii) wobei jedes von G₁, G₂ und G₃ Phenylethinyl ist, jedes von -A₁ und -A₂ -H ist, jedes von R₁ und R₃ eine hydrophile Gruppe ist und R₂ eine reaktive Gruppe ist; oder
(iii) wobei jedes von G₁, G₂ und G₃ Phenylethinyl ist, eines von -A₁ und -A₂ -H ist und das andere von -A₁ und -A₂ eine reaktive Gruppe ist, die einen Spacer beinhaltet, und jedes von R₁, R₂ und R₃ eine hydrophile Gruppe ist; oder
(iv) wobei jedes von G₁ und G₃ 4-(Carboxymethylthio)phenylethinyl ist und G₂ Phenylethinyl ist, jedes von -A₁ und -A₂ -H ist, jedes von R₁ und R₃ -H ist und R₂ eine reaktive Gruppe ist; oder
(v) wobei jedes von G₁ und G₃ 2,4-Bis(carboxymethylthio)phenylethinyl ist und G₂ Phenylethinyl ist, jedes von -A₁ und -A₂ -H ist, jedes von R₁ und R₃ -H ist und R₂ eine reaktive Gruppe ist; oder
(vi) wobei jedes von G₁ und G₃ 2,4,6-Tris(carboxymethoxy)phenylethinyl ist und G₂ Phenylethinyl ist, jedes von -A₁ und -A₂ -H ist, jedes von R₁ und R₃ -H ist und R₂ eine reaktive Gruppe ist; oder
(vii) wobei jedes von G₁ und G₃ Furan-2-yl ist und G₂ Phenylethinyl ist, jedes von -A₁ und -A₂ -H ist, jedes von R₁ und R₃ -H ist und R₂ eine reaktive Gruppe ist; oder
(viii) wobei jedes von G₁ und G₃ Thien-2-yl ist und G₂ Phenylethinyl ist, jedes von -A₁ und -A₂ -H ist, jedes von R₁ und R₃ -H ist und R₂ eine reaktive Gruppe ist; oder
(ix) wobei jedes von R₁-G₁ und R₃-G₃ (2,4-Di-(NaOOC-CH₂-O)-phenyl)ethinyl ist, G₂ (2-(NaOOC-CH₂-O)-phenyl)ethinyl ist, jedes von -A₁ und -A₂ -H ist und R₂ eine reaktive Gruppe ist.

11. Lumineszierendes Lanthanidchelat nach einem der vorhergehenden Ansprüche, wobei das Lanthanidion, Ln³⁺, aus Europium(III), Terbium(III), Dysprosium(III) und Samarium(III) ausgewählt ist, vorzugsweise Europium(III) ist.

12. Nachweisbares Molekül, das einen biospezifischen Bindungsreaktanten umfasst, der an ein wie in einem der Ansprüche 1-11 definiertes lumineszierendes Lanthanidchelat der Formel (I) konjugiert ist.

13. Nachweisbares Molekül nach Anspruch 12, wobei der biospezifische Bindungsreaktant aus einem Antikörper, einem Antigen, einem Rezeptorliganden, einem spezifischen Bindungsprotein, einer DNA-Sonde, einer RNA-Sonde, einem Oligopeptid, einem Oligonukleotid, einem modifizierten Oligonukleotid, einem modifizierten Polynukleotid, einem Protein, einem Oligosaccharid, einem Polysaccharid, einem Phospholipid, einer PNA, einem Steroid, einem Hapten, einem Wirkstoff, einem Rezeptorbindungsliganden und Lektin ausgewählt ist.

14. Nachweisbares Molekül nach einem der Ansprüche 12 oder 13, wobei der biospezifische Bindungsreaktant ein Antikörper ist.

15. Lanthanidchelatierender Ligand der Formel (II): 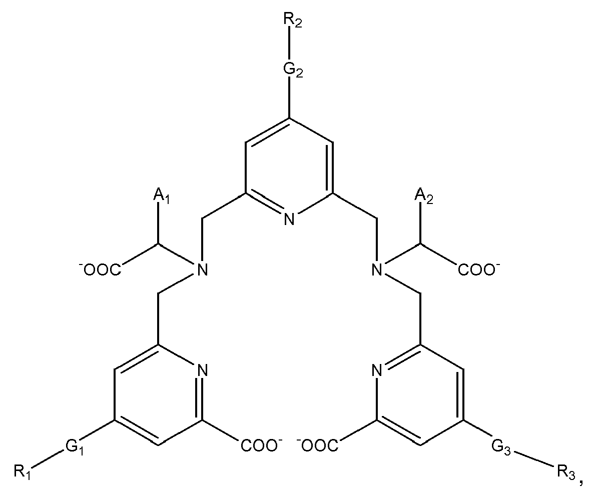 wobei jedes von G₁, G₂, G₃, R₁, R₂, R₃, A₁ und A₂ die wie in einem der Ansprüche 1-11 definierten Gruppen G₁, G₂, G₃, R₁, R₂, R₃, A₁ und A₂ darstellt.

16. Verfahren zum Durchführen eines biospezifischen Bindungsassays, wobei das Verfahren die folgenden Schritte umfasst:
a) Bilden einen Biokomplexes zwischen einem Analyt und einem biospezifischen Bindungsreaktant, der mit einem Lanthanidchelat nach einem der Ansprüche 1-11 markiert ist;
b) Anregen des Biokomplexes mit Strahlung mit einer Anregungswellenlänge, wodurch ein angeregter Biokomplex gebildet wird; und
c) Nachweisen von Emissionsstrahlung, die von dem angeregten Biokomplex emittiert wird.

17. Verwendung eines nachweisbaren Moleküls nach einem der Ansprüche 12-14 in einem spezifischen bioaffinitätsbasierten Bindungsassay unter Nutzung von zeitaufgelöster fluorometrischer Bestimmung einer spezifischen Lumineszenz.

18. Festes Trägermaterial, das mit einem lumineszenten Lanthandchelat der Formel (I) nach einem der Ansprüche 1-11 oder einem lanthanidchelatierenden Liganden der Formel (II) nach Anspruch 15 konjugiert ist.

## Revendications

1. Chélate de lanthanide luminescent répondant à la formule (I) 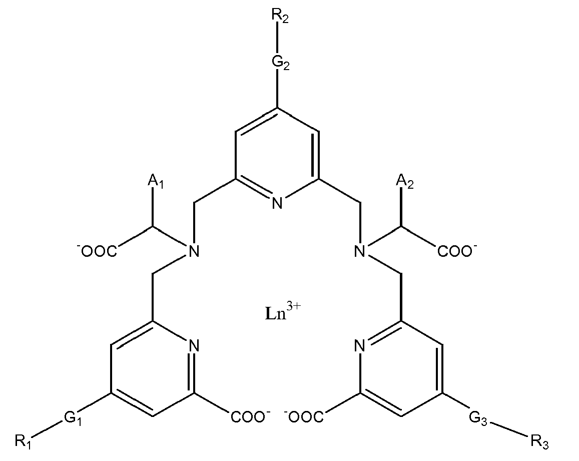 dans laquelle
chacun de G₁, G₂ et G₃ est sélectionné indépendamment parmi i) un groupe de conjugaison et ii) une liaison simple, à condition qu'au moins un parmi G₁, G₂ et G₃ soit indépendamment un groupe de conjugaison ;
chacun de R₁, R₂, et R₃ est sélectionné indépendamment parmi i) un groupe réactif Z, ii) un groupe hydrophile, and iii) l'hydrogène, ou le groupe respectif, R₁, R₂ et R₃, est absent ;
chacun de A₁ et A₂ est sélectionné indépendamment parmi i) un groupe réactif Z, ii) un groupe hydrophile, et iii) l'hydrogène ou un alkyle en C₁₋₆ ;
et dans laquelle au moins un de R₁, R₂, R₃, A₁ et A₂ est un groupe réactif Z, et
Ln³⁺ est un ion lanthanide.

2. Chélate de lanthanide luminescent selon la revendication 1, dans lequel le groupe de conjugaison consiste en une, deux ou trois fractions agencées de façon à être conjuguées entre elles, chaque fraction étant sélectionnée parmi l'éthyne (-CH=CH-), l'éthynediyle (-C≡C-), le carbonyle (-C(=O)-), les biradicaux de systèmes cycliques (hétéro)aromatiques ou de systèmes cycliques (-Het/Ar-), p. ex. le phénylène, le biphénylène, le naphtylène, le pyridylène, le pyrazinylène, le pyrimidinylène, le pyridazinylène, le furylène, le thiénylène, le pyrrolylène, l'imidazolylène, le pyrazolylène, le thiazolylène, l'isothiazolylène, l'oxazolylène, l'isoxazolylène, le fyrazanylène, le 1,2,4-triazol-3,5-ylène et l'oxadiazolylène, qui peuvent chacun être non susbstitués, monosubstitués par R₄, disubstitués par R₄,R₅, trisubstitués par R₄,R₅,R₆, tétrasubstitués par R₄,R₅,R₆,R₇ ou pentasubstitués par R₄,R₅,R₆,R₇,R₈, dans lequel chacun de tels substituants possibles R₄, R₅, R₆, R₇ et R₈ est indépendamment sélectionné parmi un alkyle en C₁₋₁₂, -(CH₂)₀₋₆COOH, -(CH₂)₀₋₆COO⁻, -(CH₂)₀₋₆SO₃H, -(CH₂)₀₋₆SO₃⁻, -NHC(=O)R₁₀, -NCH₃C(=O)R₁₀, -C(=O)NHR₁₀, -C(=O)NCH₃R₁₀,-NHC(=O)NHR₁₀, -NHC(=S)NHR₁₀, -C(=O)R₁₀, -F, -CI, -Br, -I, un hydroxyle (-OH), un groupe mercapto (-SH), -OR₉,-SR₉ et un groupe hydrophile, et dans lequel R₉ est sélectionné parmi -CF₃, un alkyle en C₁₋₁₂, -(CH₂)₁₋₆COOH, -(CH₂)₁₋₆COO⁻, -(CH₂)₁₋₆SO₃H, -(CH₂)₁₋₆SO₃⁻, -NHC(=O)R₁₀, -NCH₃C(=O)R₁₀,-C(=O)NHR₁₀, -C(=O)NCH₃R₁₀, -NHC(=O)NHR₁₀, -NHC(=S)NHR₁₀, -C(=O)R₁₀ et un groupe hydrophile, dans lequel R₁₀ est sélectionné parmi un alkyle en C₁₋₁₂,-(CH₂)₁₋₆COOH, -(CH₂)₁₋₆COO⁻, -(CH₂)₁₋₆SO₃H, et -(CH₂)₁₋₆SO₃⁻ et un groupe hydrophile.

3. Chélate de lanthanide luminescent selon l'une quelconque des revendications précédentes, dans lequel au moins deux parmi G₁, G₂ et G₃ représentent indépendamment un groupe de conjugaison.

4. Chélate de lanthanide luminescent selon l'une quelconque des revendications précédentes, dans lequel chacun parmi G₁, G₂ et G₃ représente indépendamment un groupe de conjugaison.

5. Chélate de lanthanide luminescent selon l'une quelconque des revendications précédentes, dans lequel le groupe réactif Z comprend un groupe azido (-N₃), un alcynyle (-C≡CH), un alkylène (-CH=CH₂), un groupe amino (-NH₂), un amino-oxy (-O-NH₂), un carboxyle (-COOH), un aldéhyde (-CHO), un groupe mercapto (-SH), des groupes maléimido ou leurs dérivés activés, comprenant un groupe isocyanato (-NCO), isothiocyanato (-NCS), diazonium (-N⁺N), bromoacétamido, iodoacétamido, des esters réactifs, un groupe pyridyl-2-dithio ou un groupe 4-chloro-1,3,5-triazin-2-ylamino substitué en position 6.

6. Chélate de lanthanide luminescent selon l'une quelconque des revendications précédentes, dans lequel le groupe réactif Z comprend un espaceur qui est un biradical contrôlant la distance, ledit espaceur étant formé d'une à cinq fractions, chaque fraction étant sélectionnée dans le groupe consistant en phénylène, alkylène contenant 1 à 10 atomes de carbone, un éthynediyle (-C≡C-), un éther (-O-), un thioéther (-S-), un disulfure (-S-S-), un amide (-C(=O)-NH-, -NH-C(=O)-, -C(=O)-NCH₃- et -NCH₃-C(=O)-), une thio-urée (-NH-C(=S)-NH-) et un triazole.

7. Chélate de lanthanide luminescent selon l'une quelconque des revendications précédentes, dans lequel les groupes de conjugaison sont indépendamment sélectionnés parmi le phényléthynyle, le phényle, le thiényle et le furyle, chacun pouvant être substitué.

8. Chélate de lanthanide luminescent selon l'une quelconque des revendications précédentes, dans lequel le groupe hydrophile comprend des monosaccharides ou des disaccharides.

9. Chélate de lanthanide luminescent selon l'une quelconque des revendications précédentes, dans lequel le groupe hydrophile comprend un espaceur qui est un biradical contrôlant la distance, ledit espaceur étant formé d'une à cinq fractions, chaque fraction étant sélectionnée dans le groupe consistant en phénylène, alkylène contenant 1 à 10 atomes de carbone, un éthynediyle (-C≡C-), un éther (-O-), un thioéther (-S-), un disulfure (-S-S-), un amide (-C(=O)-NH-, -NH-C(=O)-, -C(=O)-NCH₃- et -NCH₃-C(=O)-), une thio-urée (-NH-C(=S)-NH-) et un triazole.

10. Chélate de lanthanide luminescent selon l'une quelconque des revendications précédentes
(i) dans lequel chacun de G₁ et G₃ est le 3,5-bis(carboxyméthoxy)phényléthynyle et G₂ est le phényléthynyle, chacun de -A₁ et -A₂ est -H, chacun de R₁ et R₃ est -H, et R₂ est un groupe réactif ; ou
(ii) dans lequel chacun de G₁, G₂ et G₃ est le phényléthynyle, chacun de -A₁ et-A₂ est -H, chacun de R₁ et R₃ est un groupe hydrophile, et R₂ est un groupe réactif ; ou
(iii) dans lequel chacun de G₁, G₂ et G₃ est le phényléthynyle, un de -A₁ et -A₂ est -H, et l'autre de -A₁ et -A₂ est un groupe réactif comprenant un espaceur, et chacun de R₁, R₂ et R₃ est un groupe hydrophile ; ou
(iv) dans lequel chacun de G₁ et G₃ et le 4-(carboxyméthylthio)phényléthynyle et G₂ est le phényléthynyle, chacun de -A₁ et -A₂ est -H, chacun de R₁ et R₃ est -H, et R₂ est un groupe réactif ; ou
(v) dans lequel chacun de G₁ et G₃ est le 2,4-bis(carboxyméthylthio)phényléthynyle et G₂ est le phényléthynyle, chacun de - A₁ et -A₂ est -H, chacun de R₁ et R₃ est -H, et R₂ est un groupe réactif ; ou
(vi) dans lequel chacun de G₁ et G₃ est le 2,4,6-tris(carboxyméthoxy)-phényléthynyle et G₂ est le phényléthynyle, chacun de -A₁ et -A₂ est -H, chacun de R₁ et R₃ est -H, et R₂ est un groupe réactif ; ou
(vii) dans lequel chacun de G₁ et G₃ est le furan-2-yle et G₂ est le phényléthynyle, chacun de -A₁ et -A₂ est -H, chacun de R₁ et R₃ est -H, et R₂ est un groupe réactif ; ou
(viii) dans lequel chacun de G₁ et G₃ est le thién-2-yle et G₂ est le phényléthynyle, chacun de -A₁ et -A₂ est -H, chacun de R₁ et R₃ est -H, et R₂ est un groupe réactif ; ou
(ix) dans lequel chacun de R₁-G₁ et R₃-G₃ est le (2,4-di(NaOOC-CH₂-O-)phényl)éthynyle, G₂ est le (2-(NaOOC-CH₂-O-)phényl)éthynyle, chacun de -A₁ et -A₂ est -H, et R₂ est un groupe réactif.

11. Chélate de lanthanide luminescent selon l'une quelconque des revendications précédentes, dans lequel l'ion lanthanide, Ln³⁺, est sélectionné parmi l'europium(III), le terbium(III), le dysprosium(III) et le samarium(III), de préférence l'europium(III).

12. Molécule détectable comprenant un réactif de liaison biospécifique conjugué à un chélate de lanthanide luminescent répondant à la formule (I) tel que défini selon l'une quelconque des revendications 1 à 11.

13. Molécule détectable selon la revendication 12, dans laquelle le réactif de liaison biospécifique est sélectionné parmi un anticorps, un antigène, un ligand de récepteur, une protéine de liaison spécifique, une sonde ADN, une sonde ARN, un oligopeptide, un oligonucléotide, un oligonucléotide modifié, un polynucléotide modifié, une protéine, un oligosaccaride, un polysaccharide, un phospholipide, un APN, un stéroïde, un haptène, un médicament, un ligand de liaison à un récepteur, et une lectine.

14. Molécule détectable selon l'une quelconque des revendications 12 ou 13, dans laquelle le réactif de liaison biospécifique est un anticorps.

15. Ligand de chélation d'un lanthanide répondant à la formule (II), 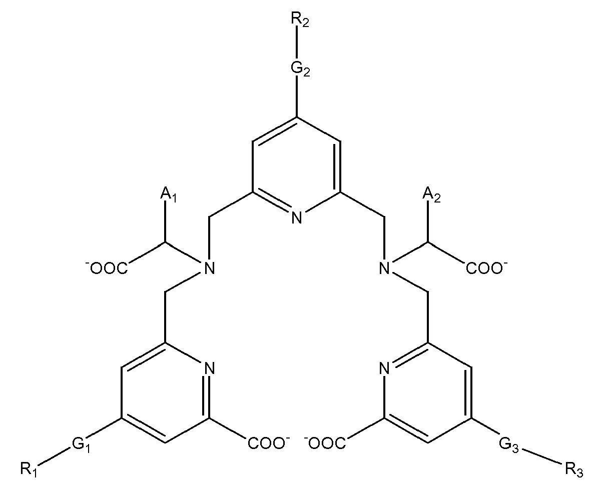 dans laquelle chacun de G₁, G₂, G₃, R₁, R₂, R₃, A₁ et A₂ représente les groupes G₁, G₂, G₃, R₁, R₂, R₃, A₁ et A₂ tels que définis selon l'une quelconque des revendications 1 à 11.

16. Procédé de réalisation d'un dosage de liaison biospécifique, ledit procédé comprenant les étapes consistant à :
a) former un biocomplexe entre un analyte et un réactif de liaison biospécifique marqué par un chélate de lanthanide selon l'une quelconque des revendications 1 à 11 ;
b) exciter ledit biocomplexe avec un rayonnement présentant une longueur d'onde d'excitation, ce qui permet de former un biocomplexe excité ; et
c) détecter le rayonnement d'émission émis par ledit biocomplexe excité.

17. Utilisation d'une molécule détectable selon l'une quelconque des revendications 12 à 14 dans un dosage de liaison basé sur une bioaffinité spécifique à l'aide de la détermination fluorimétrique en temps résolu d'une luminescence spécifique.

18. Matériau de support solide conjugué à un chélate de lanthanide luminescent répondant à la formule (I) selon l'une quelconque des revendications 1 à 11 ou ligand de chélation de lanthanide répondant à la formule (II) selon la revendication 15.
